# EUROPEAN PATENT SPECIFICATION

(11) **EP 0 923 582 B1**
(45) Date of publication and mention of the grant of the patent: **20.09.2006**
(21) Application number: 97930697.4
(22) Date of filing: 25.07.1997
(51) Int. Cl.: C07D 487/04, C07D 471/04, A61K 31/505, A61K 31/435, C07D 239/00, C07D 231/00, C07D 249/00, C07D 221/00, C07D 209/00

(54) **SUBSTITUTED 6,5-HETERO-BICYCLIC DERIVATIVES**
SUBSTITUIERTE 6,5-HETEROBICYCLISCHE-DERIVATE
DERIVES 6,5-HETERO-BICYCLIQUES SUBSTITUES

(30) Priority: 28.08.1996 US 25039 P
(43) Date of publication of application: 23.06.1999
(73) Proprietor: PFIZER INC., New York, N.Y. 10017 (US)
(72) Inventor: CHEN, Yuhpyng, Liang, Waterford, CT 06385 (US)
(74) Representative: O'Brien, Niall James
(86) International application number: PCT/IB1997/000922
(87) International publication number: WO 1998/008847

(56) References cited:
- WO-A-95/33750
- WO-A-96/35689

## Description

### Background of the invention

This invention relates to certain pharmaceutically active substituted 6,5-heterobicyclic derivatives, pharmaceutical compositions containing them and methods of administering them to subjects in need of their corticotropin releasing factor antagonist activity.

The substituted heterocyclic derivatives claimed in this case exhibit activity as corticotropin releasing factor (hormone) CRF (CRH) antagonists.

CRF antagonists are mentioned in U.S. Patents 4,605,642 and 5,063,245 referring to peptides and pyrazolinones, respectively. They are also referred to in the following: PCT Patent Application PCT/IB95/00439, which designates the United States and was filed on June 6, 1995 and published on December 14, 1995; PCT Patent Application PCT/IB95/00373, which designates the United States and was filed on May 18, 1995 and published on December 21, 1995; U.S. Patent Application 08/448,539, which was filed in the PCT on Nov. 12, 1993 and entered the U.S. national phase on June 14, 1995; PCT Patent Application WO 95/10506, which was filed on October 12, 1993 and published on April 20, 1995, and U.S. Patent Application 08/481,413, which was filed in the PCT on November 26, 1993 and entered the U.S. national phase on July 24, 1995; U.S. Patent Application 08/254,820, which was filed on April 19, 1995; Provisional U.S. Patent Application 60/008,396, which was filed on December 8, 1995; and Provisional U.S. Patent Application 60/006,333, which was filed on November 8, 1995.

The importance of CRF antagonists is set out in the literature, e.g., P. Black, Scientific American SCIENCE & MEDICINE, 1995, p. 16-25; T. Lovenberg et al., Current Pharmaceutical Design, 1995, 1, 305-316; and United States Patent 5,063,245, which is referred to above. A recent outline of the different activities possessed by CRF antagonists is found in M. J. Owens et al., Pharm. Rev., Vol. 43, pages 425 to 473 (1991). Based on the research described in these two and other references, CRF antagonists are effective in the treatment of a wide range of stress-related illnesses, mood disorders such as depression, major depressive disorder, single episode depression, recurrent depression, child abuse induced depression, postpartum depression, dysthemia, bipolar disorders and cyclothymia; chronic fatigue syndrome; eating disorders such as anorexia and bulimia nervosa; generalized anxiety disorder, panic disorder, phobias; obsessive-compulsive disorder, post-traumatic stress disorder, pain perception such as fibromyalgia; headache; gastrointestinal diseases; hemorrhagic stress; ulcers; stress-induced psychotic episodes; fever; diarrhea; post-operative ileus, colonic hypersensitivity; irritable bowel syndrome; Crohn's disease; spastic colon; inflammatory disorders such as rheumatoid arthritis and osteoarthritis; pain; asthma; psoriasis; allergies; osteoporosis; premature birth; hypertension, congestive heart failure; sleep disorders; neurodegenerative diseases such as Alzheimer's disease, senile dementia of the Alzheimer's type, multiinfarct dementia, Parkinson's disease, and Huntington's disease; head trauma; ischemic neuronal damage; excitotoxic neuronal damage; epilepsy; stroke: spinal cord trauma; psychosocial dwarfism; euthyroid sick syndrome; syndrome of inappropriate antidiarrhetic hormone; obesity; chemical dependencies and addictions; drug and alcohol withdrawal symptoms; infertility, cancer; infertility; muscular spasms; urinary incontinence; hypoglycemia and immune dysfunctions including stress induced immune dysfunctions, immune suppression and human immunodeficiency virus infections; and stress-induced infections in humans and animals.

The compounds of this invention are also believed to be inhibitors of CRH binding protein and therefore useful in the treatment of disorders the treatment of which can be effected or facilitated by inhibiting such protein. Examples of such disorders are Alzheimer's disease and obesity.

### Summary of the Invention

The present invention relates to compounds of the formula 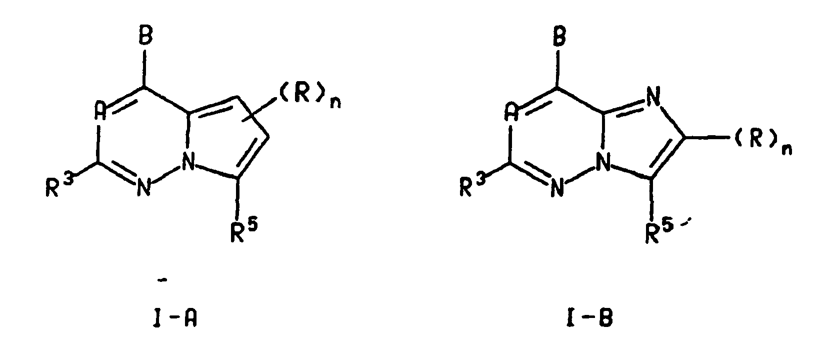 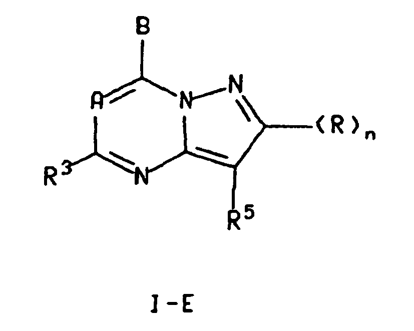 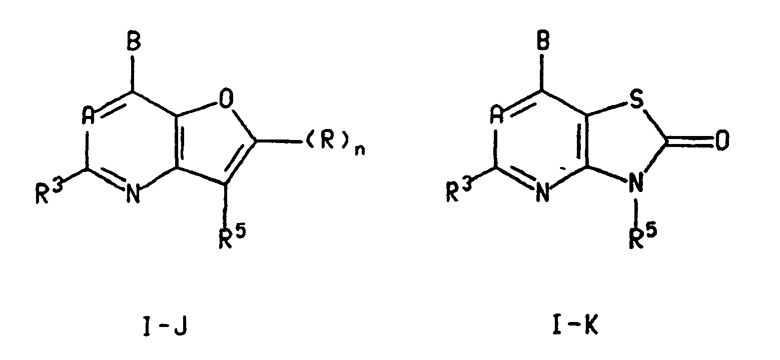 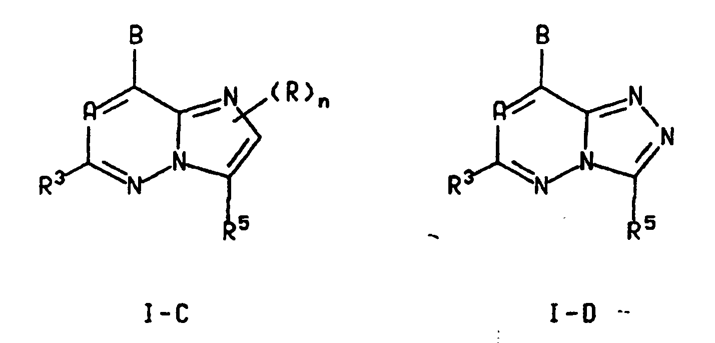 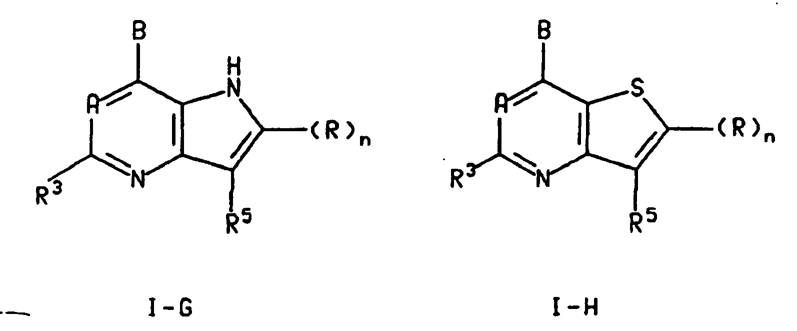 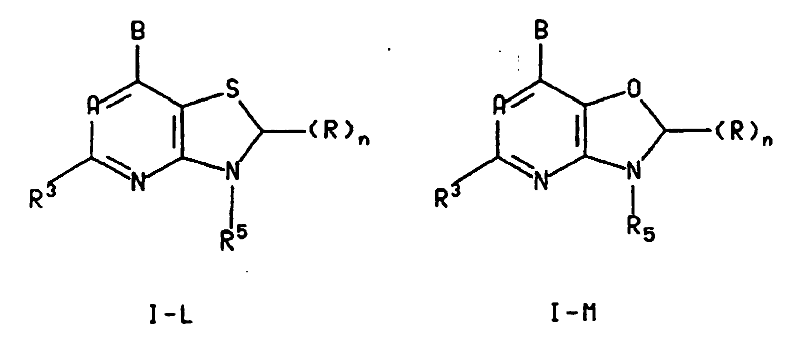 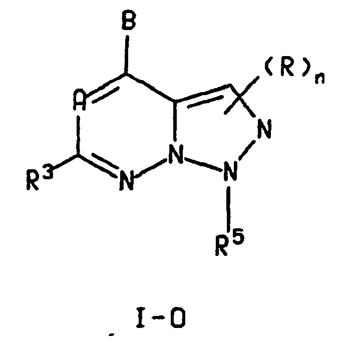 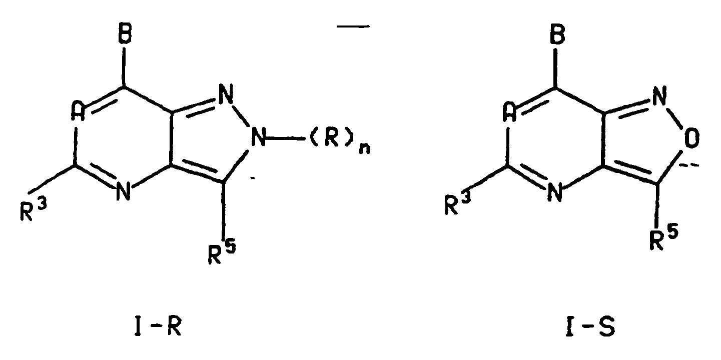 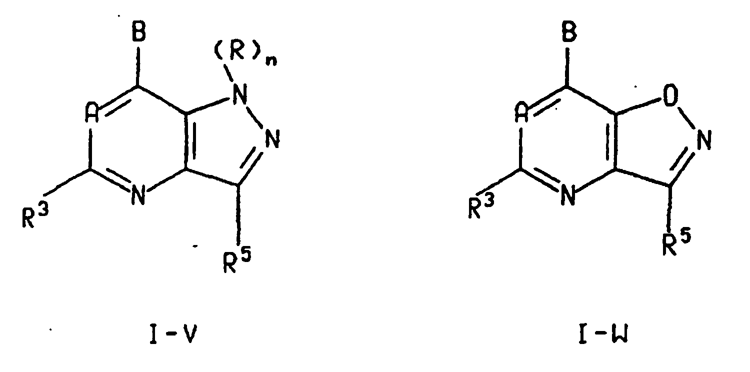 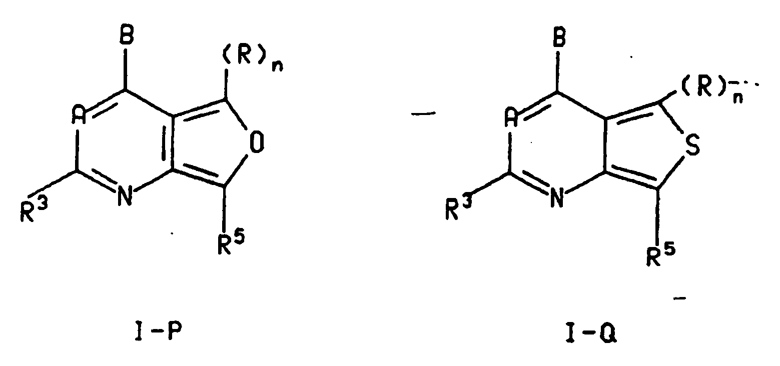 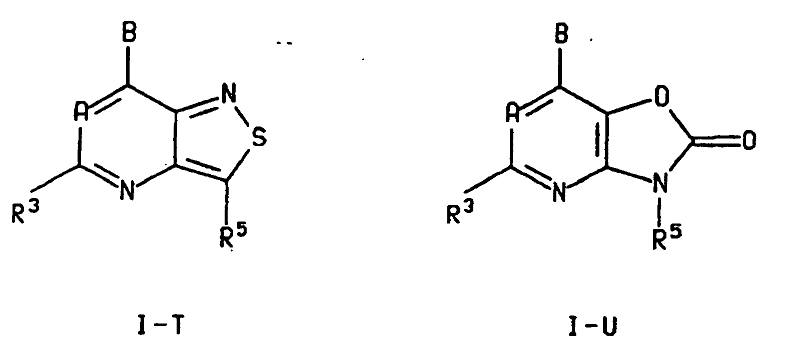 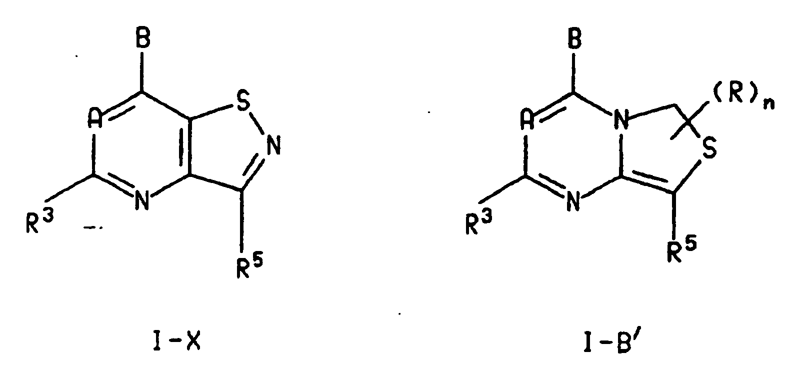 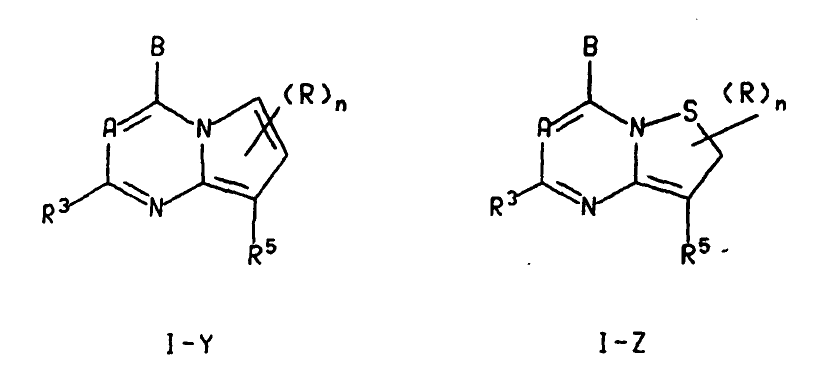 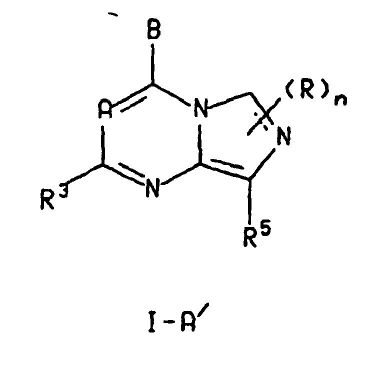 wherein
A is nitrogen or CR⁷;
B is -CHR¹R², -NR¹R², -NHCHR¹R²,-OCHR¹R² or -SCHR¹R²;
R¹ is C₁-C₆ alkyl optionally substituted with one hydroxy, fluoro, CF₃, or C₁-C₄ alkoxy group, wherein the C₁-C₄ alkoxy group may optionally contain one double or triple bond;
R² is benzyl or C₁-C₆ alkyl which may optionally contain one double or triple bond, and wherein said C₁-C₆ alkyl and the phenyl moiety of said benzyl may optionally be substituted with one fluoro, CF₃, C₁-C₂ alkyl, C₁-C₂ alkoxy or chloro group;
R³ is hydrogen, C₁-C₄ alkyl, -O(C₁-C₄ alkyl), chloro, fluoro, bromo, iodo, (C₁-C₂ alkylene)-O-(C₁-C₂ alkyl), (C₁-C₂ alkylene)-OH or -S(C₁-C₄ alkyl);
R⁵ is phenyl, pyridyl, pyrazinyl, pyrimdil, pyridazinyl and wherein each of the foregoing R⁵ groups is substituted with from one to four substituents R¹³ wherein one to three of said substituents may be selected, independently, from fluoro, chloro, C₁-C₆ alkyl and -O(C₁-C₆ alkyl) and one of said substituents may be selected from bromo, iodo, formyl, OH, (C₁-C₄ alkylene)-OH, -CN, -CF₃, -NO₂, -NH₂, -NH(C₁-C₄ alkyl), -N(C₁-C₂ alkyl)(C₁₋C₆ alkyl), -OCO(C₁-C₄ alkyl), (C₁-C₄ alkylene)-O-(C₁-C₄ alkyl), -S(C₁-C₆ alkyl), (C₁-C₄ alkylene)-S-(C₁-C₄alkyl), -C(=O)O(C₁-C₄ alkyl), -C(=O)(C₁-C₄ alkyl), -COOH,-SO₂NH(C₁-C₄alkyl), -SO₂N(C₁-C₂-alkyl)(C₁-C₄alkyl), -SO₂NH₂, -NHSO₂(C₁-C₄ alkyl),-S(C₁-C₆ alkyl) and -SO₂(C₁-C₆ alkyl), and wherein each of the C₁-C₄ alkyl and C₁-C₆ alkyl moieties in the foregoing R⁵ groups may optionally have one or two double bonds;
R⁷ is hydrogen, C₁-C₄ alkyl, halo (e.g., chloro, fluoro, iodo or bromo), hydroxy,-O(C₁-C₄ alkyl), -C(=O)(C₁-C₄ alkyl),-C(=O)O(C₁-C₄ alkyl), -OCF₃, -CF₃, -CH₂OH or-CH₂O(C₁-C₂ alkyl);
(R)ₙ represents from zero to two substituents, wherein when n is 1 and said (R)₁ group is attached to a carbon atom, (R)₁ is hydrogen, (C₁-C₆) alkyl, fluoro, chloro, bromo, iodo, hydroxy, cyano, amino, (C₁-C₂ alkylene)-OH, CF₃, CH₂SCH₃, nitro, O(C₁-C₄ alkyl),-N(C₁-C₄ alkyl)(C₁-C₂ alkyl), -S(C₁-C₄ alkyl), -CO(C₁-C₄ alkyl), -COH or -COO(C₁-C₄ alkyl); and wherein when n is 1 and said (R)₁ is attached to a nitrogen atom, (R)₁ is hydrogen or C₁-C₄ alkyl; and wherein when n is 2 and (R)₂ is attached to a carbon atom, (R)₂ is R^{a}R^{b}, wherein R^{a} is hydrogen, C₁-C₆ alkyl, fluoro, chloro, bromo; iodo, hydroxy, cyano, amino, (C₁-C₂ alkylene)-OH, CF₃, CH₂SCH₃, nitro, -O(C₁-C₄ alkyl), -N(C₁-C₄ alkyl)(C₁-C₂ alkyl), -S(C₁-C₄ alkyl), -CO(C₁-C₄ alkyl), -COH or -COO(C₁-C₄ alkyl); and R^{b} is hydrogen, methyl or ethyl;
and the pharmaceutically acceptable salts of such compounds.

The term "alkyl", as used herein, unless otherwise indicated, includes saturated monovalent hydrocarbon radicals having straight branched or cyclic moieties or combinations thereof.

The term "alkoxy", as used herein, unless otherwise indicated, means -O-alkyl, where "alkyl" is defined as above.

More specific embodiments of this invention include compounds of the above formula I wherein B is -NR¹R², -NHCHR¹R² or -OCHR¹R², and R¹ is C₁-C₆ alkyl, which may optionally be substituted with one fluoro or C₁-C₄ alkoxy group and which may optionally contain one double or triple bond; and R² is C₁-C₄ alkyl which may optionally contain one double or triple bond.

Other more specific embodiments of this invention include compounds of formula I wherein R³ is methyl, ethyl, chloro or methoxy; wherein when (R)₁ is attached to a carbon atom or (R)₂ is attached to a carbon atom, (R)₁, (R)₂, R^{a} and R^{b} are selected from hydrogen, methyl and ethyl; R⁵ is di- or tri-substituted phenyl, pyridyl, or pyrimidyl, in which the two or three substitutents on said phenyl, pyridyl or pyrimidyl are selected, independently, from fluoro or chloro C₁-C₄ alkyl, -O-(C₁-C₄ alkyl), and one of the two to three may be selected from (C₁-C₄alkyl)-O-(C₁-C₄ alkyl), -CF₃, -OCF₃, -CHO, -(C₁-C₄ alkyl)-OH, cyano, bromo and iodo, wherein each of the forgoing C₁-C₄ alkyl groups may optionally contain one double or triple bond;

Other more specific embodiments of this invention include compounds of the formula I wherein A is nitrogen or CR⁷ and R⁷ is hydrogen or methyl.

Examples of preferred compounds of the formula
7-(1-ethyl-propoxy)-5-methyl-3-(2,4,6-trimethyl-phenyl)-pyrazolo[1,5-a]pyrimidine;
[2,5-dimethyl-3-(2,4,6-trimethyl)-pyrazolo[1,5-a]pyrimidin-7yl]-(1-ethylpropyl)-amine;
(1-ethyl-propyl)-[5-methyl-3-(2,4,6-trimethyl)-pyrazolo[1,5-a]pyrimidin-7-yl]amine;
7-(1-ethyl-propoxy)-2,5-dimethyl-3-(2,4,6-trimethyl-phenyl)-pyrazolo[1,5-a]pyrimidine;
[2,5-dimethyl-3-(2,4,6-trimethyl-phenyl)-pyrazolo[1,5-a]pyrimidin-7-yl]-ethyl-propylamine;

Other compounds of formula I include the following:
4-(1-ethyl-propoxy)-2,7-dimethyl-8-(2,4,6-trimethyl-phenyl)-pyrrolo[1,2-a]pyrimidine;
(1-ethyl-propyl)-[5-methyl-3-(2,4,6-trimethyl-phenyl)-pyrazolo[1,5-a]pyrimidin-7-yl]amine;
[3-(4-bromo-2,6-dimethyl-phenyl)-5-methyl-pyrazolo[1,5-a]pyrimidin-7-yl]-(1-ethylpropyl)-amine;
butyl-ethyl-[5-methyl-3-(2,4,6-trimethyl-phenyl)-pyrazolo[1,5-a]pyrimidin-7-yl]amine;
3-(4-bromo-2,6-dimethyl-phenyl)-7-(1-ethyl-propoxy)-5-methyl-pyrazolo[1,5-a]pyrimidine;
[2,7-dimethyl-8-(2,4,6-trimethyl-phenyl)pyrrolo[1,2-a]pyrimidin-4-yl]-diethyl-amine;
[2,7-dimethyl-8-(2,4,6-trimethyl-phenyl)-pyrrolo[1,2-a]pyrimidin-4-yl]-ethyl-propyl-amine;
[8-(4-chloro-2,6-dimethyl-phenyl)-2,7-dimethyl-pyrrolo[1,2-a]pyrimidin-4-yl]-ethyl-propyl-amine;
[8-(4-chloro-2,6-dimethyl-phenyl)-2,7-dimethyl-pyrrolo[1,2-a]pyrimidin-4-yl]-diethyl-amine;
[8-(4-chloro-2,6-dimethyl-phenyl)-2,6-dimethyl-pyrrolo[1,2-a]pyrimidin-4-yl]-diethyl-amine;
[2,6-dimethyl-8-(2,4,6-trimethy)-phenyl)-pyrrolo[1,2-a]pyrimidin-4-yl]-diethyl-amine;
[2,6-dimethyl-8-(2,4,6-trimethyl-phenyl)-pyrrolo[1,2-a]pyrimidin-4-yl]-ethyl-propyl-amine;
butyl-[2,6-dimethyl-8-(2,4,6-trimethyl-phenyl)-pyrrolo[1,2-a]pyrimidin-4-yl]-ethyl-amine;
[2,6-dimethyl-8-(2,4,6-trimethyl-phenyl)-pyrrolo[1,2-a]pyrimidin-4-yl]-(1-ethyl-propyl)-amine;
[8-(4-chloro-2,6-dimethyl-phenyl)-2,6-dimethyl-pyrrolo[1,2-a]pyrimidin-4-yl]-(1-ethyl-propyl)-amine;
8-(4-chloro-2,6-dimethyl-phenyl)-4-(1-ethyl-propoxy)-2,6-dimethyl-pyrrolo[1,2-a]pyrimidine;
4-(1-ethyl-propoxy)-2,6-dimethyl-8-(2,4,6-trimethyl-phenyl)pyrrolo[1,2-a]pyrimidine;
4-(1-ethyl-propoxy)-2-methyl-8-(2,4,6-trimethyl-phenyl)-pyrrolo[1,2-a]pyrimidine;
(1-ethyl-propyl)-[2-methyl-8-(2,4,6-trimethyl-phenyl)-pyrrolo[1,2-a]pyrimidin-4-yl]-amine;
butyl-ethyl-[2-methyl-8-(2,4,6-trimethyl-phenyl)-pyrrolo[1,2-a]pyrimidin-4-yl]-amine;
butyl-ethyl-[5-methyl-3-(2,4,6-trimethyl-phenyl)-2H-isothiazolo[2,3-a]pyrimidin-7-yl]-amine;
(1-ethyl-propyl)-[5-methyl-3-(2,4,6-trimethyl-phenyl)-2H-isothiazolo [2,3-a]pyrimidin-7-yl]-amine;
7-(1-ethyl-propoxy)-5-methyl-3-(2,4,6-trimethyl-phenyl)-2H-isothiazolo[2,3-a]pyrimidine;
4-(1-ethyl-propoxy)-2-methyl-8-(2,4,6-trimethyl-phenyl)-imidazo[1,5-a]pyrimidine;
(1-ethyl-propyl)-[2-methyl-8-(2,4,6-trimethyl-phenyl)-imidazo[1,5-a]pyrimidin-4-yl]-amine;
[2,6-dimethyl-8-(2,4,6-trimethyl-phenyl)-imidazo[1,5-a]pyrimidin-4-yl]-(1-ethylpropyl)-amine;
4-(1-ethyl-propoxy)-2,6-dimethyl-8-(2,4,6-trimethyl-phenyl)-imidazo[1,5-a]pyrimidine;
4-(1-ethyl-propoxy)-2,6-dimethyl-8-(2,4,6-trimethyl-phenyl)-6,7-dihydro-imidazo [1,5-a]pyrimidine;
4-(1-ethyl-propoxy)-2,6-dimethyl-8-(2,4,6-trimethyl-phenyl)-thiazolo[3,4-a]pyrimidine;
4-(1-ethyl-propoxy)-2-methyl-7-(2,4,6-trimethyl-phenyl)-pyrrolo[1,2-b]pyridazine;
4-(1-ethyl-propoxy)-2,5-dimethyl-7-(2,4,6-trimethyl-phenyl)-pyrrolo[1,2-b]pyridazine;
4-(1-ethyl-propoxy)-2,6-dimethyl-7-(2,4,6-trimethyl-phenyl)-pyrrolo[1,2-b]; pyridazine;
4-(1-ethyl-propoxy)-2,5,6-trimethyl-7-(2,4,6-trimethyl-phenyl)-pyrrolo[1,2-b]pyridazine;
[2,5-dimethyl-7-(2,4,6-trimethyl-phenyl)-pyrrolo[1,2-b]pyridazin-4-yl]-(1-ethyl-propyl)-amine;
(1-ethyl-propyl)-[2-methyl-7-(2,4,6-trimethyl-phenyl)-pyrrolo[1,2-b]pyridazin-4-yl]-amine;
[2,6-dimethyl-3-(2,4,6-trimethyl-phenyl)-imidazo[1,2-b]pyridazin-8-yl]-(1-ethyl-propyl)-amine;
8-(1-ethyl-propoxy)-2,6-dimethyl-3-(2,4,6-trimethyl-phenyl)-imidazo[1,2-b]pyridazine;
8-(1-ethyl-propoxy)-6-methyl-3-(2,4,6-trimethyl-phenyl)-imidazo[1,2-b]pyridazine;
(1-ethyl-propyl)-[6-methyl-3-(2,4,6-trimethyl-phenyl)-imidazo[1,2-b]pyridazin-8-yl]-amine;
(1-ethyl-propyl)-[2-methyl-7-(2,4,6-trimethyl-phenyl)-imidazo [1,5-b]pyridazin-4-yl]-amine;
[2,5-dimethyl-7-(2,4,6-trimethyl-phenyl)-imidazo[1,5-b]pyridazin-4-yl]-(1-ethyl-propyl)-amine;
4-(1-ethyl-propoxy)-2,5-dimethyl-7-(2,4,6-trimethyl-phenyl)-imidazo[1,5-b]pyridazine-b]pyridazine;
butyl-[2,5-dimethyl-7-(2,4,6-trimethyl-phenyl)-imidazo[1,5-b]pyridazin-4-yl]-ethyl-amine;
[2,5-dimethyl-7-(2,4,6-trimethyl-phenyl)-imidazo[1,5-b]pyridazin-4-yl]-ethyl-propyl-amine;
[2,5-dimethyl-7-(2,4,6-trimethyl-phenyl)-imidazo[1,5-b]pyridazin-4-yl]-diethyl-amine;
diethyl-[6-methyl-3-(2,4,6-trimethyl-phenyl)-[1,2,4]triazolo[4,3-b]pyridazin-8-yl]-amine;
ethyl-[6-methyl-3-(2,4,6-trimethyl-phenyl)-[1,2,4]triazolo[4,3-b]pyridazin-8-yl]-propyl-amine;
butyl-ethyl-[6-methyl-3-(2,4,6-trimethyl-phenyl)-[1,2,4]triazolo[4,3-b]pyridazin-8-yl] amine;
(1-ethyl-propyl)-[6-methyl-3-(2,4,6-trimethyl-phenyl)-[1,2,4]triazolo[4,3-b]pyridazin-8-yl]-amine;
8-(1-ethyl-propoxy)-6-methyl-3-(2,4,6-trimethyl-phenyl)-[1,2,4]triazolo[4,3-b]pyridazine;
7-(1-ethyl-propoxy)-1,5-dimethyl-3-(2,4,6-trimethyl-phenyl)-1H-pyrrolo[3,2-b]pyridine;
7-(1-ethyl-propoxy)-1,2,5-trimethyl-3-(2,4,6-trimethyl-phenyl)-1H-pyrrolo[3,2-b]pyridine;
(1-ethyl-propyl)-[1,2,5-trimethyl-3-(2,4,6-trimethyl-phenyl)-1H-pyrrolo[3,2-b]pyridin-7-yl]-amine;
[1,5-dimethyl-3-(2,4,6-trimethyl-phenyl)-1H-pyrrolo[3,2-b]pyridin-7-yl]-(1-ethyl-propyl)-amine;
butyl-[1,5-dimethyl-3-(2,4,6-trimethyl-phenyl)-1H-pyrrolo[3,2-b]pyridin-7-yl]-ethyl-amine;
[1,5-dimethyl-3-(2,4,6-trimethyl-phenyl)-1H-pyrrolo[3,2-b]pyridin-7-yl]-ethyl-propyl-amine;
[1,5-dimethyl-3-(2,4,6-trimethyl-phenyl)-1H-pyrrolo[3,2-b]pyridin-7-yl]-diethyl-amine;
7-(1-ethyl-propoxy)-5-methyl-3-(2,4,6-trimethyl-phenyl)-isothiazolo[4,5-b]pyridine;
(1-ethyl-propyl)-[5-methyl-3-(2,4,6-trimethyl-phenyl)-isothiazolo[4,5-b]pyridin-7-yl]-amine;
(1-ethyl-propyl)-[5-methyl-3-(2,4,6-trimethyl-phenyl)-isoxazolo[4,5-b]pyridin-7-yl]-amine;
7-(1-ethyl-propoxy)-5-methyl-3-(2,4,6-trimethyl-phenyl)-isoxazolo[4,5-b]pyridine;
7-(1-ethyl-propoxy)-5-methyl-3-(2,4,6-trimethyl-phenyl)-thieno[3,2-b]pyridine;
7-(1-ethyl-propoxy)-2,5-dimethyl-3-(2,4,6-trimethyl-phenyl)-thieno[3,2-b]pyridine;
[2,5-dimethyl-3-(2,4,6-trimethyl-phenyl)-thieno[3,2-b]pyridin-7-yl]-(1-ethyl-propyl)-amine;
(1-ethyl-propyl)-[5-methyl-3-(2,4,6-trimethyl-phenyl)-thieno[3,2-b]pyridin-7-yl]-amine;
(1-ethyl-propyl)-[5-methyl-3-(2,4,6-trimethyl-phenyl)-furo[3,2-b]pyridin-7-yl]-amine;
[2,5-dimethyl-3-(2,4,6-trimethyl-phenyl)-furo[3,2-b]pyridin-7-yl]-(1-ethyl-propyl)-amine;
7-(1-ethyl-propoxy)-2,5-dimethyl-3-(2,4,6-trimethyl-phenyl)-furo[3,2-b]pyridine;
7-(1-ethyl-propoxy)- 5-methyl-3-(2,4,6-trimethyl-phenyl)-furo[3,2-b]pyridine;
4-(1-ethyl-propoxy)-2,5-dimethyl-7-(2,4,6-trimethyl-phenyl)-6H-pyrrolo[3,4-b]pyridine;
4-(1-ethyl-propoxy)-2,5,6-trimethyl-7-(2,4,6-trimethyl-phenyl)-6H-pyrrolo[3,4-b]pyridine;
(1-ethyl-propyl)-[2,5,6-trimethyl-7-(2,4,6-trimethyl-phenyl)-6H-pyrrolo[3,4-b]pyridin-4-yl]-amine;
[1,5-dimethyl-3-(2,4,6-trimethyl-phenyl)-2-thia-4-aza-inden-7-yl]-(1-ethyl-propyl)-amine;
7-(1-ethyl-propoxy)-1,5-dimethyl-3-(2,4,6-trimethyl-phenyl)-2 thia-4-aza-indene;
4-(1-ethyl-propoxy)-2,5-dimethyl-7-(2,4,6-trimethyl-phenyl)-furo[3,4-b]pyridine;
[2,5-dimethyl-7-(2,4,6-trimethyl-phenyl)-furo[3,4-b]pyridin-4-yl]-(1-ethyl-propyl)-amine;
[2,5-dimethyl-3-(2,4,6-trimethyl-phenyl)-2H-pyrazolo[4,3-b]pyridin-7-yl]-(1-ethyl-propyl)-amine;
7-(1-ethyl-propoxy)-2,5-dimethyl-3-(2,4,6-trimethyl-phenyl)-2H-pyrazolo[4,3-b]pyridine;
7-(1-ethyl-propoxy)-5-methyl-3-(2,4,6-trimethyl-phenyl)-isothiazolo[4,3-b]pyridine;
(1-ethyl-propyl)-[5-methyl-3-(2,4,6-trimethyl-phenyl)-isothiazolo[4,3-b]pyridin-7-yl]-amine;
[2,5-dimethyl-3-(2,4,6-trimethyl-phenyl)-pyrazolo[1,5-a]pyrimidin-7-yl]-diethyl-amine;
butyl-[2,5-dimethyl-3-(2,4,6-trimethyl-phenyl)-pyrazolo[1,5-a]pyrimidin-7-yl]-ethyl-amine-,
butyl-ethyl-[5-methyl-3-(2,4,6-trimethyl-phenyl)-pyrazolo[1,5-a]pyrimidin-7-yl]-amine;
ethyl-[5-methyl-3-(2,4,6-trimethyl-phenyl)-pyrazolo[1,5-a]pyrimidin-7-yl]-propyl-amine;
diethyl-[5-methyl-3-(2,4,6-trimethyl-phenyl)-pyrazolo[1,5-a]pyrimidin-7-yl]-amine;
[3-(4-chloro-2,6-dimethyl-phenyl)-5-methyl-pyrazolo [1,5-a]pyrimidin-7-yl]-(1 - ethyl-propyl)-amine;
[3-(4-chloro-2,6-dimethyl-phenyl)-5-methyl-pyrazolo[1,5-a]pyrimidin-7-yl]-(1-ethyl-propyl)-amine;
3-(4-chloro-2,6-dimethyl-phenyl)-7-(1-ethyl-propoxy)-5-methyl-pyrazolo[1,5-a]pyrimidine;
8-(4-chloro-2,6-dimethyl-phenyl)-4-(1-ethyl-propoxy)-2-methyl-pyrazolo[1,5-a][1,3,5]triazine;
(1-ethyl-propyl)-[2-methyl-8-(2,4,6-trimethyl-phenyl)-pyrazolo[1,5-a][1,3,5]triazin-4-yl]-amine;
4-(1-ethyl-propoxy)-2-methyl-8-(2,4,6-trimethyl-phenyl)-pyrazolo[1,5-a][1,3,5]triazine;
4-(1-ethyl-propoxy)-2,7-dimethyl-8-(2,4,6-trimethyl-phenyl)-pyrazolo[1,5-a][1,3,5]triazine;
[2,7-dimethyl-8-(2,4,6-trimethyl-phenyl)-pyrazolo[1,5-a][1,3,5]triazin-4-yl]-(1-ethyl-propyl)-amine;
[1,5-dimethyl-3-(2,4,6-trimethyl-phenyl)-1H-pyrazolo[4,3-b]pyridin-7-yl]-(1-ethyl-propyl)-amine;
7-(1-ethyl-propoxy)-1,5-dimethyl-3-(2,4,6-trimethyl-phenyl)-1H-pyrazolo[4,3-b]pyridine;
7-(1-ethyl-propoxy)-5-methyl-3-(2,4,6-trimethyl-phenyl)-isothiazolo[4,5-b]pyridine;
(1-ethyl-propyl)-[5-methyl-3-(2,4,6-trimethyl-phenyl)-isothiazolo[4,5-b]pyridin-7-yl]-amine;
1,5-dimethyl-3-(2,4,6-trimethyl-phenyl)-7-(1-ethyl-propoxy)-1H-pyrrolo[3,2-b]pyridine;
1,5-dimethyl-3-(2,6-dimethyl-4-chloro-phenyl)-7-(1-ethyl-propoxy)-1H-pyrrolo[3,2-b]pyridine;
2,5-dimethyl-3-(2,4,6-trimethyl-phenyl)-7-(1-ethyl-propoxy)-1H-pyrrolo[3,2-b]pyridine;
2,5-dimethyl-3-(2,6-dimethyl-4-chloro-phenyl)-7-(1-ethyl-propoxy)-1H-pyrrolo[3,2-b]pyridine;
7-(1-cyclopropylmethyl-propoxy)-1,5-dimethyl-3-(2,4,6-trimethyl-phenyl)-1H-pyrrolo[3,2-b]pyridine;
7-(1-cyclopropylmethyl-propoxy)-1,5-dimethyl-3-(2,6-dimethyl-4-chloro-phenyl)-1H-pyrrolo[3,2-b]pyridine;
1,5-dimethyl-3-(2,4,6-trimethyl-phenyl)-7-(tetrahydro-furan-3-yloxy)-1H-pyrrolo[3,2-b]pyridine;
1,5-dimethyl-3-(2,4,6-trimethyl-phenyl)-7-(S)-(tetrahydro-furan-3-yloxy)-1H-pyrrolo[3,2-b]pyridine;
1,5-dimethyl-3-(2,4,6-trimethyl-phenyl)-7-(1-propyl-butoxy)-1H-pyrrolo[3,2-b]pyridine;
7-sec-butylsulfanyl-1,5-dimethyl-3-(2,4,6-trimethyl-phenyl)-1H-pyrrolo[3,2-b]pyridine;
cyclopropylmethyl-[2,5-dimethyl-7-(2,4,6-trimethyl-phenyl)-5H-pyrrolo[3,2-b]pyrimidin-4-yl]-propyl-amine;
2-[1,5-dimethyl-3-(2,4,6-trimethyl-phenyl)-1H-pyrrolo[3,2-b]pyridin-7-ylamino]-butan-1-ol;
1,5-dimethyl-7-thiazolidin-3-yl-3-(2,4,6-trimethyl-phenyl)-1H-pyrrolo[3,2-b]pyridine;
[1,5-dimethyl-3-(2,4,6-trimethyl-phenyl)-1H-pyrrolo[3,2-b]pyridin-7-yl]-ethyl-(2,2,2-trifluoro-ethyl)-amine;
cyclopropylmethyl-[1,5-dimethyl-3-(2,4,6-trimethyl-phenyl)-1H-pyrrolo[3,2-b]pyridin-7-yl]-propyl-amine;
(1-ethyl-propyl)-[5-methyl-3-(2,4,6-trimethyl-phenyl)-isoxazolo[4,5-b]pyridin-7-yl]-amine; and
7-(1-ethyl-propoxy)-5-methyl-3-(2,4,6-trimethyl-phenyl)-isoxazolo[4,5-b]pyridine**.**

This invention also relates to a pharmaceutical composition for the treatment, prevention or inhibition of (a) a disorder the treatment of which can be effected or facilitated by antagonizing CRF, including but not limited to disorders induced or facilitated by CRF, or (b) a disorder selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome, Crohn's disease; spastic colon; post operative ileus; ulcer, diarrhea; stress-induced fever, human immunodeficiency virus (HIV) infections; neurodegenerative diseases such as Alzheimer's disease, Parkinson's disease and Huntington's disease; gastrointestinal diseases; eating disorders such as anorexia and bulimia nervosa; hemorrhagic stress; chemical dependencies and addictions (e.g., dependencies on alcohol, nicotine, cocaine, heroin, benzodiazepines, or other drugs); drug and alcohol withdrawal symptoms; stress-induced psychotic episodes; euthyroid sick syndrome; syndrome of inappropriate antidiuretic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions (e.g., porcine stress syndrome, bovine shipping fever, equine paroxysmal fibrillation, and dysfunctions Induced by confinement in chickens, sheering stress in sheep or human-animal interaction related stress in dogs); muscular spasms; urinary incontinence; senile dementia of the Alzheimer's type; multiinfract dementia; amyotrophic lateral sclerosis; hypertension; tachycardia; congestive heart failure; osteoporosis; premature birth; and hypoglycemia in a mammal, including a human, comprising an amount of a compound of the formula I, or a pharmaceutically acceptable salt thereof, that is effective in the treatment of such disorder, and a pharmaceutically acceptable carrier.

The invention also relates to the use of a compound of formula I or a pharmaceutically acceptable salt thereof for the preparation of a medicament for the treatment, prevention or inhibition of a disorder selected from inflammatory disorders such as rheumatoid arthritis and osteoarthritis, pain, asthma, psoriasis and allergies; generalized anxiety disorder, panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome; Crohn's disease; spastic colon; post operative ileus; ulcer, diarrhea; stress-inducedfever; human immunodeficiency virus (HIV) infections; neurodegenerative diseases such as Alzheimer's disease, Parkinson's disease and Huntington's disease; gastrointestinal diseases; eating disorders such as anorexia and bulimia nervosa; hemorrhagic stress; stress-induced psychotic episodes; euthyroid sick syndrome; syndrome of inappropriate antidiuretic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions (e.g., porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; chemical dependencies and addictions (e.g., dependencies on alcohol, nicotine, cocaine, heroin, benzodiazepines, or other drugs); drug and alcohol withdrawal symptoms; hypertension; tachycardia; congestive heart failure; osteoporosis; premature birth; and hypoglycemia in a mammal, including a human, comprising administering to a subject in need of said treatment an amount of a compound of the formula I, or a pharmaceutically acceptable salt thereof, that is effective in treating such disorder.

This invention also relates to a pharmaceutical composition for treating or preventing a disorder or conditon, the treatment or prevention of which can be effected or facilitated by inhibiting CRH binding protein in a mammal, including a human, comprising a CRH binding protein inhibiting amount of a compound of the formula I, or a pharmaceutically acceptable salt thereof, and a pharmaceutically acceptable carrier.

This invention includes all optical isomers and other stereoisomers of compounds of the formula I. When such compounds contain one or more chiral centers, it is understood that the invention includes the racemic mixtures as well as all individual enantiomers and diastereomers of such compounds, and mixtures thereof.

### Detailed Description of the Invention

The following compounds having the formulas II, III, IV and V are useful as intermediates in the synthesis of compounds of the formula I. 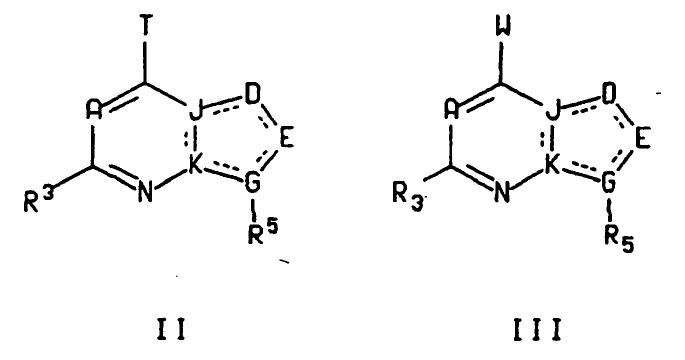 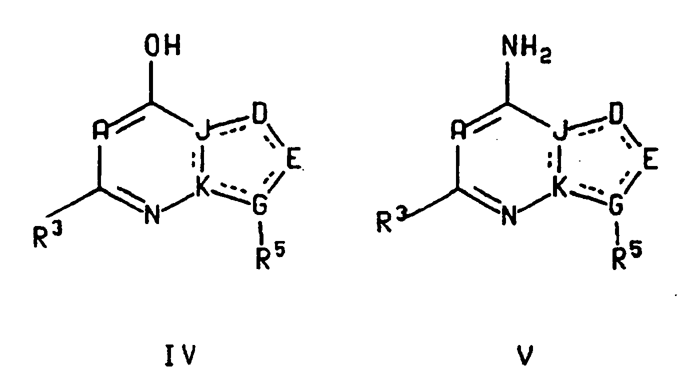 wherein T is chloro, bromo, iodo or -OSO₂CF₃; W is cyano, formyl, or -COO(C₀-C₄ alkyl) and A, J, K, D, E, G, R³, and R⁵ are as defined above with reference to formula I.

Compounds of the formula I may be prepared as described below. In the reaction schemes and discussion that follow, A, B, D, E, G, J, K, R³, R⁵ and structural formulae I, II, III, IV, and V are defined as above.

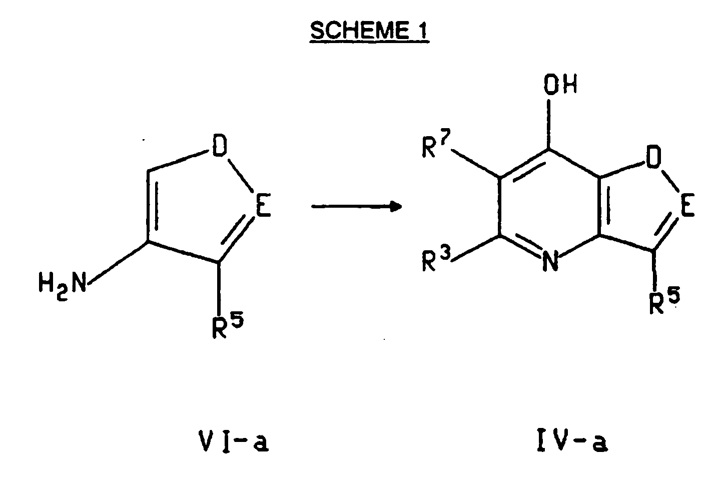

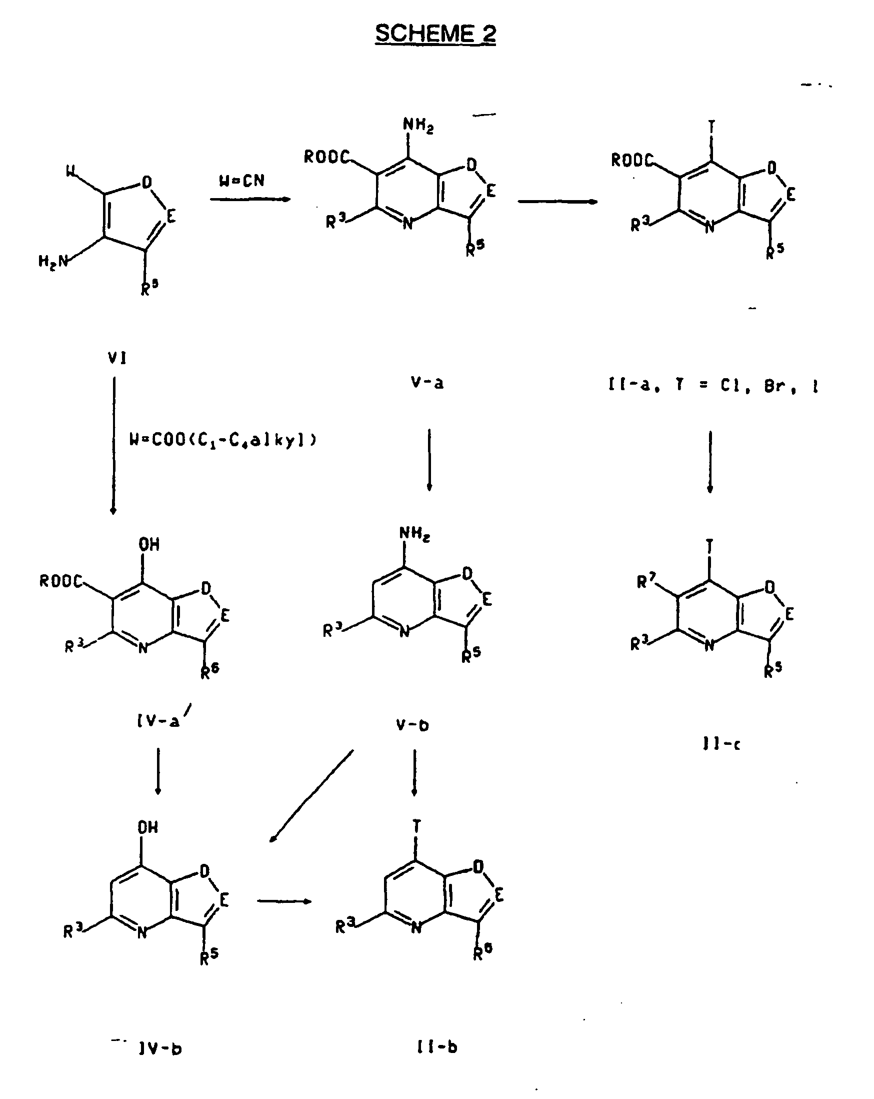

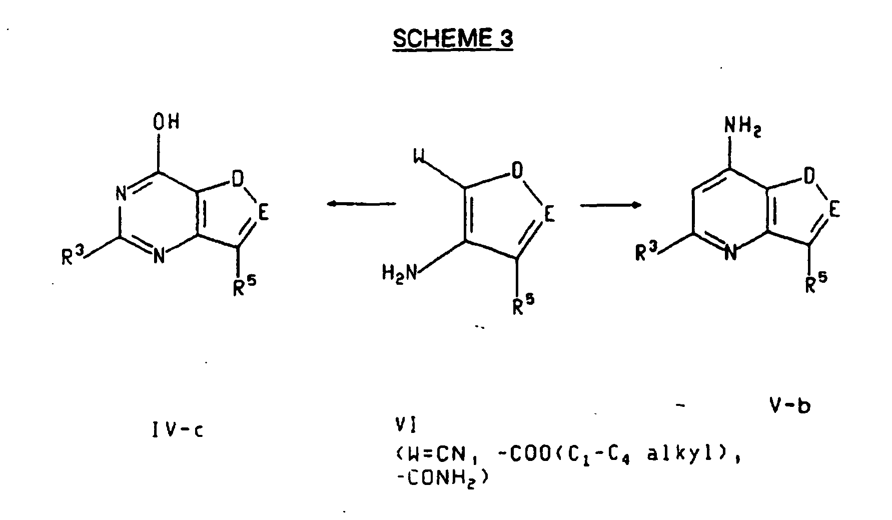

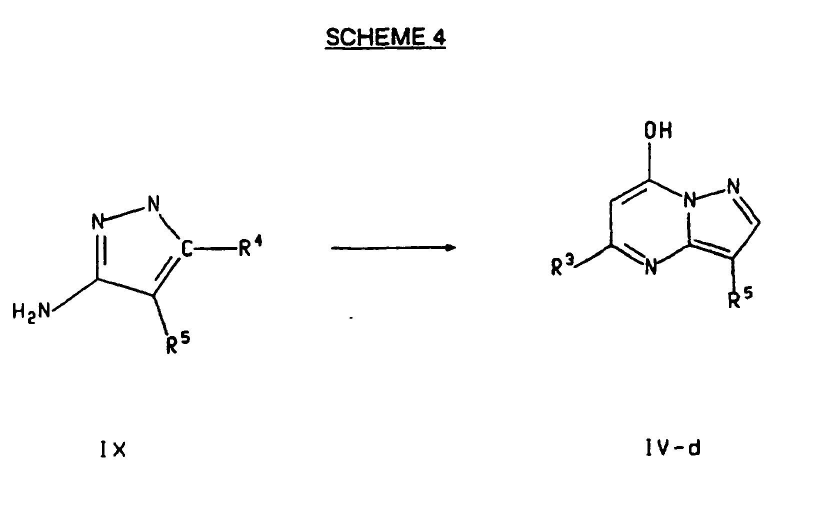

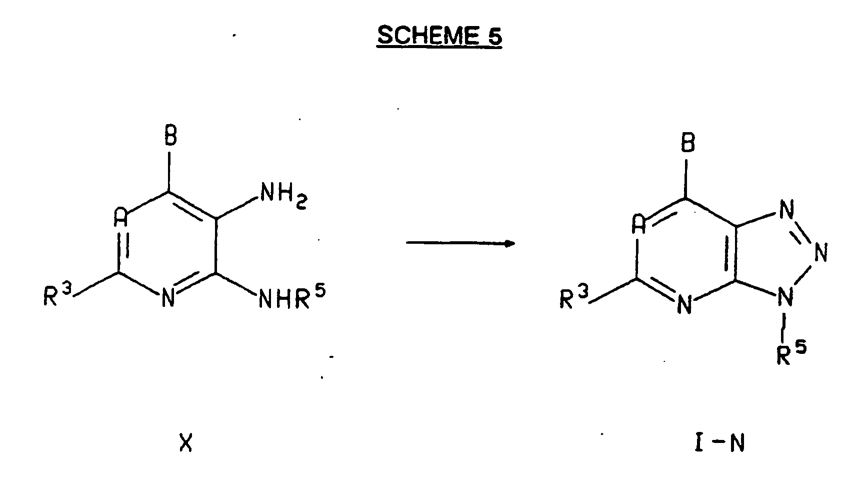

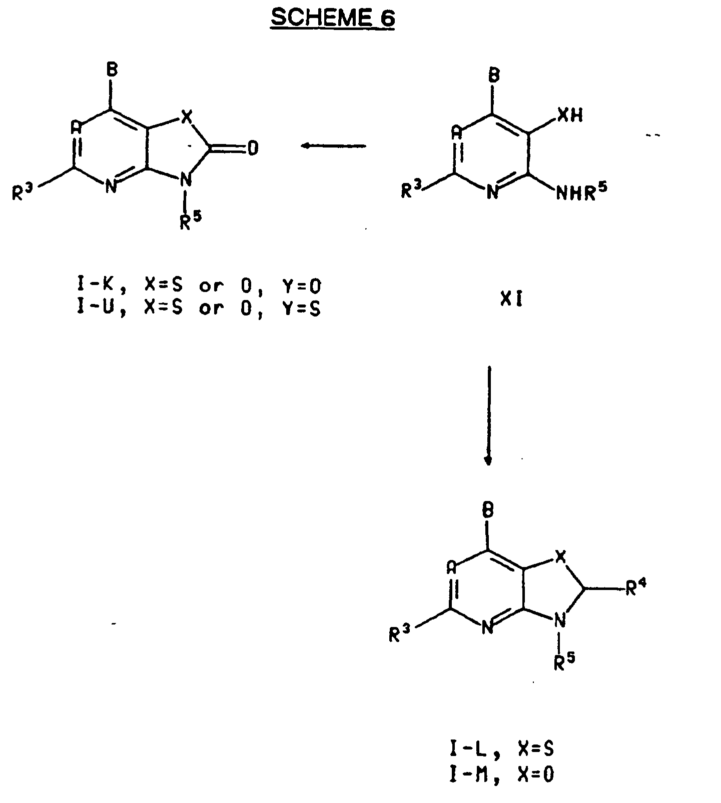

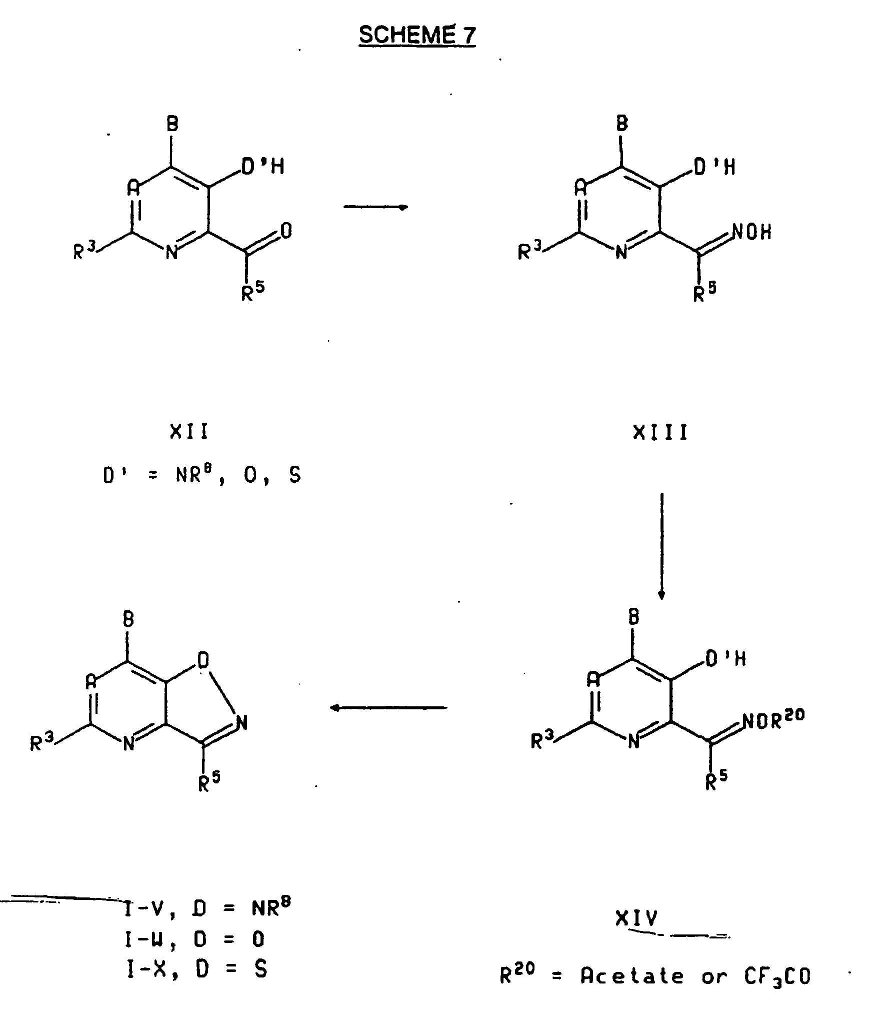

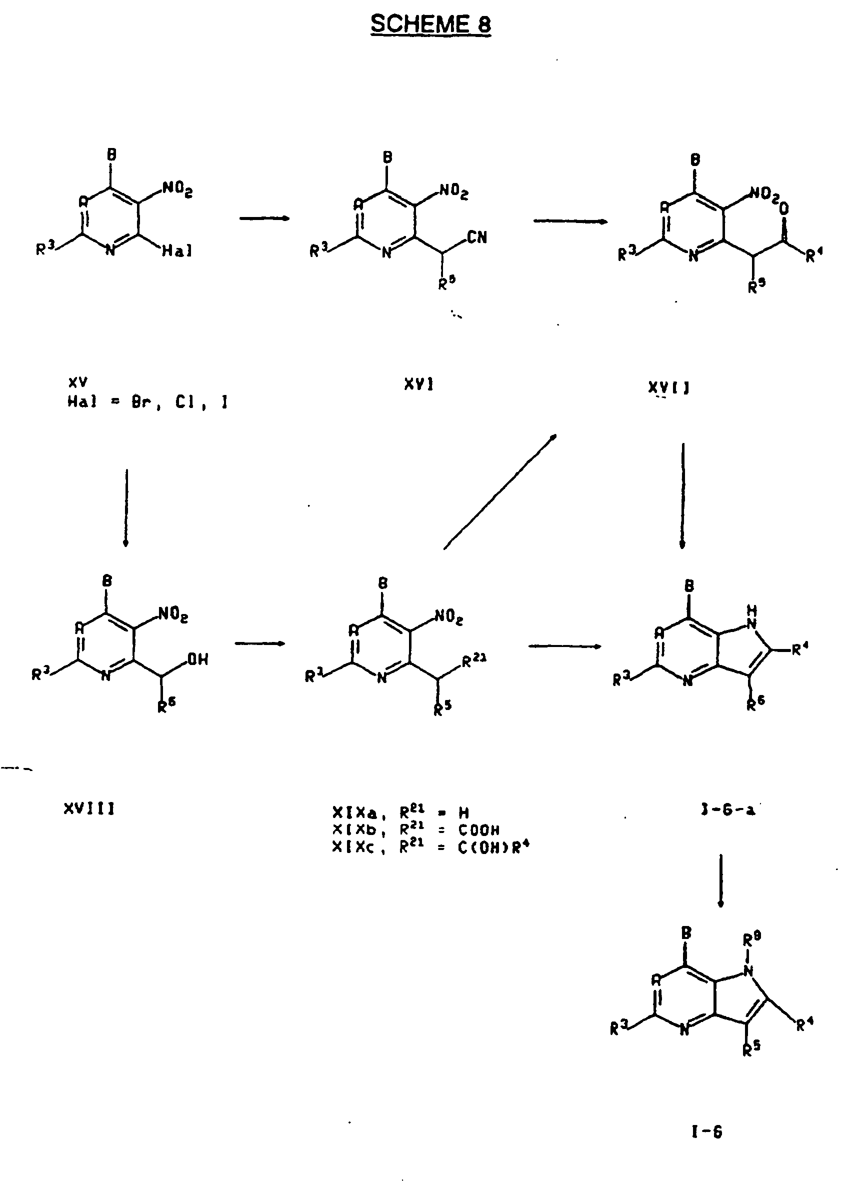

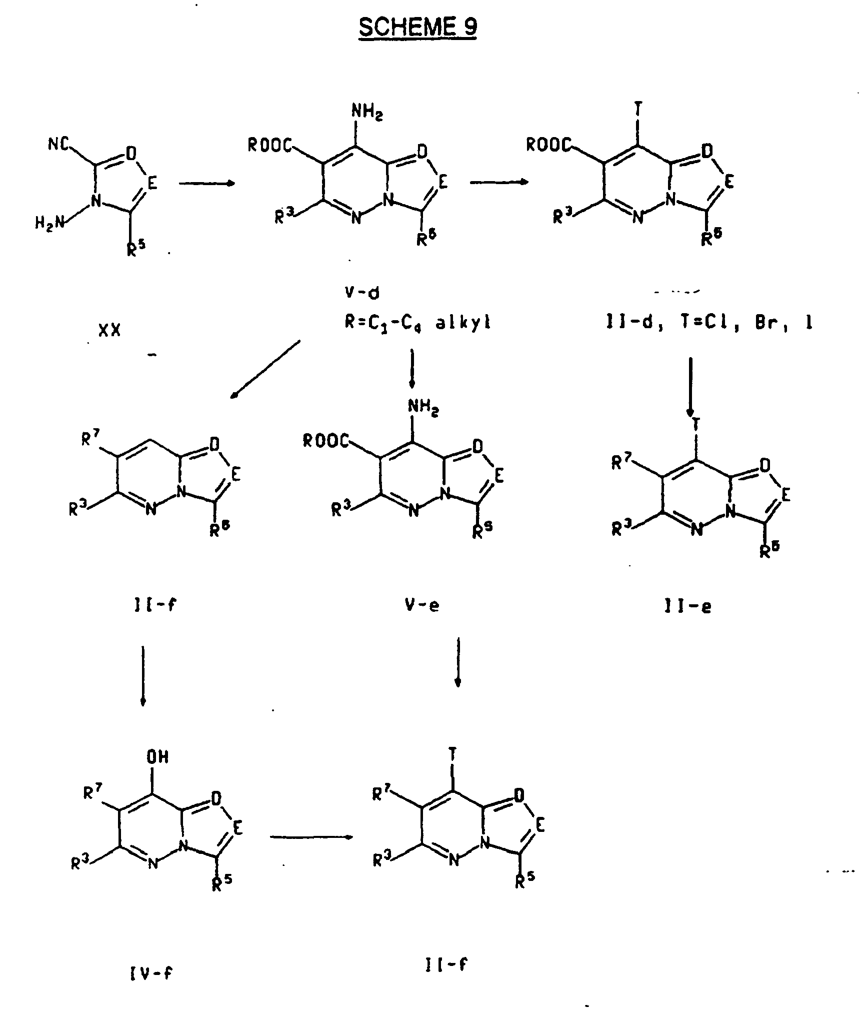

Compounds of the formula I wherein B is -NR¹R² or -NHCR¹R²R¹¹ may be prepared by reacting a compound of the formula II wherein T is chloro, bromo, or iodo with a compound of the formula BH, in the presence of a base, with or without an organometallic compound such as Cu(I)X, wherein X is chloro, bromo or iodo, or an acid (such as p-TsOH (Ts=Tosyl) or another sterically hindered phenol) or an equivalent agent known to those of skill in the art. Suitable solvents for this reaction include DMSO, NMP, dimethylacetamide and THF. An excess of BH may be used as both the reagent and the base. Other bases such as potassium or sodium carbonate, a trialkylamine, a potassium or sodium (C₁-C₄ alkoxide) and sodium hydride may also be used. When R⁷ is an electron withdrawing group such as -COO(C₁-C₄alkyl) or CN, the reaction generally is carried out at a temperature between about room temperature and about 100°C. When R⁷ is a non-electron withdrawing group, the reaction temperature can generally range from about 50°C to about 270°C and the pressure can generally range from about 4 psi to about 300 psi. A pressure reactor may be used.

Alternatively, the compounds of formula I may be prepared by reacting a compound of the formula II wherein T is bromo or iodo with 1 equivalent or an excess of BH and a base such as sodium or potassium carbonate or a sodium or potassium (C₁-C₄ alkoxide), in the presence of a palladium (II) or a palladium (O) catalyst such as Pd(OAc)₂ or Pd(PPh₃)₄, together with a racemic or chiral phosphino agent such as 2,2-bis(diphenylphosphino)-1,1-binaphthyl (BINAP). Alternatively, premade Pd(II)(BINAP) may be used in an appropriate inert (i.e., inert with respect to the reaction at hand) solvent such as toluene, xylene, dioxane or sulfolane, at a temperature from about room temperature to about 180°C, preferably at about reflux temperature.

Compounds of the formula I wherein B is -OCR¹R²R¹¹, -SCR¹R²R¹¹, or -NHCR¹R²R¹¹ may be prepared by reacting compounds in the formula II wherein T is chloro, bromo or iodo with a compound of the formula BH in the presence of a base which is capable of deprotonation of BH (e.g., sodium or potassium hydride, or an organometallic base such as sodium diisopropylamide, sodium bis(trimethylsilyl)amide, lithium diisopropylamide, lithium bis(trimethylsilyl)amide, a sodium C₁-C₄ alkoxide or n-butyllithium), in an inert organic solvent such as tetrahydrofuran, acetonitrile, dimethylsulfoxide, acetone, a C₂-C₅ alcohol, chloroform, benzene, xylene, toluene, N,N-dimethylformamide (DMF), methylene chloride, 1-methyl-2-pyrrolidinone or a mixture of two or more of the above solvents (e.g., DMSO and THF), at a temperature from about 0°C to about 180°C, preferably from about 50°C to about 180°C.

Compounds of the formula I wherein B is -CR¹R²R¹¹, -C(C=CR²R¹²)R¹, -CR²R¹¹NHR¹, -CR²R¹¹OR¹, -CR²R¹¹SR¹, or -C(O)R² may be prepared from compounds of the formula III wherein W is cyano, formyl or carboxy, as described below.

Reacting compounds of formula III wherein W is cyano with a Grignard reagent containing the group R² in an inert solvent such as THF, glyme, ether or dioxane, will yield corresponding compounds of formula I wherein B is -COR². Further reaction of the compounds of formula I wherein B is COR² with a Grignard reagent containing R¹, using a solvent such as those referred to above, will yield the corresponding the compounds of formula I wherein B is -CR¹R²OH. Reacting compounds of formula III wherein W is formyl with a Grignard reagent containing the group R², in an ethereal solvent such as those referred to above, will yield the corresponding compounds of formula I wherein B is -CHR²OH.

Compounds of formula I wherein B is -CR¹R²R¹¹ or -C(C=CR²R¹¹)R¹ may be prepared by conventional methods. Thus, reaction of a compound of the formula I wherein B is -CR^{1'}R^{2'}OH, (wherein R^{1'} and R^{2'} are defined as R¹ and R², respectively, except that R^{1'} may not be R¹ and R^{2'} may not be R²), with an acid such as concentrated sulfuric acid in acetic acid, or a Burgess inner salt such as (carboxysulfamoyl)triethylammonium hydroxide methyl ester, will yield a compound of the formula I wherein B is -C(=CR²R¹¹)R¹. Hydrogenation of a compound of formula I wherein B is -C(=CR²R¹¹)R¹ using palladium on carbon (Pd/C) or a platinum dioxide catalyst in a (C₁-C₄)alkanol solvent will yield a compound of the formula I wherein B is -CHR¹R². Reaction of a compound of the formula I wherein B is -CR¹R²OH with diethylaminosulfur trifluoride or triphenylphosphine/carbon tetrachloride in an inert solvent such as carbon tetrachloride will afford a compound of the formula I wherein B is -CR¹R²F or -CR¹R²Cl, respectively.

Reduction of a compound of formula I wherein B is -COR² with sodium borohydride, in an inert solvent such as a (C₁-C₄)alkanol, will yield a compound of the formula I wherein B is -CHR²OH. Alkylation of a compound of the formula I wherein B is -CHR²OH with an alkyl halide (such as alkyl iodide) in the presence of a base such as sodium hydride (NaH) at room temperature, in an inert solvent such as toluene, THF, dioxane or ether, will yield the corresponding compound of the formula I wherein B is -CHR²OR¹.

Compounds of the formula I wherein B is -CR²A¹⁰NHR¹ may be prepared by conventional methods such as reductive amination of the corresponding compounds of the formula I wherein B is -C(O)R² with an appropriate amine and reducing agent (such as sodium cyanoborohydride, sodium triacetoxyborohydride, or lithium aluminum tetrahydride) in an appropriate inert solvent such as a C₁-C₄ alkanol or acetic acid.

Conversion of compounds in formula I wherein B is -C(O)R² into compounds in formula I wherein B is -C(S)R² can be accomplished using standard methods well known in the art (e.g., using Lawesson's Reagent or diphosphorus pentasulfide (P₂S₅)). Reduction of compounds of the formula I wherein B is -C(S)R² with a reducing agent such as sodium borohydride or lithium aluminum tetrahydride gives the corresponding compounds of the formula I wherein B is -CHR²SH. Alkylation of compounds of the formula I wherein B is -CHR²SH with alkyl halide (such as alkyl iodide) in the presence of a base such as sodium hydride in an inert solvent such as THF, DMF or toluene at about room temperature will afford the corresponding compounds of the formula I wherein B is -CHR²SR¹.

Compounds in formula II may be prepared from compounds of the formula IV or V as described below.

Compounds of formula II wherein T is chloro, bromo or iodo can be prepared by reacting compounds of the formula IV with from one equivalent to an excess of POT₃ (wherein T is chloro, bromo or iodo) in the presence or absence of a di(C₁-C₄ alkyl)aniline, preferably diethylaniline, with or without a solvent (such as dichloroethane, DMF, dimethylsulfoxide (DMSO) or acetamide), at a temperature from about room temperature to about 180°C, preferably from about 100°C to about 150°C. Alternatively, compounds of formula II wherein T is chloro, bromo or iodo can be prepared by reacting the corresponding compounds of formula II wherein T is -O-SO₂CF₃ with a sodium or potassium halide, in an appropriate inert solvent such as THF, sulfolane, DMSO, DMF or acetonitrile, at a temperature from about 60°C to about 180°C Compounds of formula II wherein T is -OSO₂CF₃ can be prepared by reacting compounds of formula IV with Tf₂O in the presence of a base such as triethylamine or pyridine, in an appropriate inert solvent such as THF, methylene chloride, dioxane, ether or toluene, at an temperature from about 0°C to about 50°C, preferably from about 0°C to about room temperature.

Alternatively, compounds of formula II wherein T is chloro, bromo or iodo may be prepared by reacting compounds of formula V with a (C₁-C₇ alkyl)-nitrite and Cu(I)T₂ (wherein T is chloro, bromo or iodo) in an appropriate inert solvent such as acetonitrile, acetone, methylene chloride, THF, dioxane, benzene, toluene, dichloroethans, DMF, DMSO or N-methylpyrrolidinone (NMP) at a temperature from about room temperature to about 150°C, preferably from about 40°C to about 100°C.

Compounds of the formula IV may be prepared by reacting the appropriate compounds of formula V with sodium nitrite (NaNO₂) in an aqueous acid such as sulfuric acid, acetic acid or phosporic acid, with or without an organic solvent, preferably in acetonitrite (CH₃CN) or acetone.

Compounds of formula III wherein W is cyano can be prepared by reacting the corresponding compounds of formula II wherein T is chloro, bromo or iodo with potassium cyanide, copper cyanide, sodium cyanide or a di(C₁-C₄alkyl)aluminum cyanide in an appropriate inert solvent such as dimethylsulfoxide. DMF, toluene or xylene, at temperature from about room temperature to about 180°C, preferably from about 60°C to about 150°C, with or without Pd(II)OAc or Pd(0)(PPh₃)₄.

Compounds of formula III wherein W is -CHO or -COOH may be prepared by reacting compounds in formula II wherein T is bromo or iodo with an organolithium reagent such as t-BuLi, s-BuLi, or n-BuLi in an appropriate inert solvent such as THF, dioxane, ether, benzene or methylene chloride, at temperature from about -120°C to about room temperature, preferably from about -110°C to about -60°C, followed by quenching with an appropriate electrophile such as DMF or CO₂ (gas or dry ice), to give compounds of formula III wherein W is -CHO and -COOH, respectively.

It is understood that the general organic chemistry knowledge can be applied to change the steps of the reaction sequences described herein depending on the feasibility of the reaction. For example, a protecting group may be used at any stage of the various syntheses described above at which it is workable, or an ester group may be reduced to the corresponding C₁-C₄ alkyl group at any convenient stage. Compounds of formulas I - XVIII, wherein R³ is chloro, bromo, -COO(C₁-C₄ alkyl) or -COOH, can be converted to the corresponding compounds wherein R³ is (C₁-C₄ alkyl), -O(C₁-C₄ alkyl), fluoro, -S(C₁-C₄ alkyl) at any convenient stage, as appropriate, in the syntheses referred to above using methods described in the literature. Compounds of formula I - XVIII, wherein R³ is -O(C₁-C₄ alkyl) or -S(C₁-C₄ alkyl) can be prepared by reacting the corresponding compounds wherein R³ is chloro, bromo or iodo, with a nucleophile such as a C₁-C₄ alkanol or a C₁-C₄ alkanethiol, in the presence of an organic or inorganic base. Suitable bases for such a reaction include sodium and sodium hydride. Compounds of formulas I - XVIII wherein R³ is fluoro can be prepared by reacting the corresponding compounds wherein R³ is chloro with tetrabutylammonium fluoride in a suitable inert solvent such as DMSO, methylene chloride, or tetrahydrofuran. Tetrahydrofuran is preferred.

Reduction of an ester or carboxylic acid using lithium aluminum tetrahydride/aluminum trichloride (LiAlH₄/AlCl₃) in an appropriate inert solvent such as THF, ether, or dioxane, at temperature from about 100°C to about room temperature, will afford the corresponding compound wherein R³ is CH₃. Conversion of compounds wherein B is -COOH to the corresponding compounds wherein B is -CO(C₁-C₃ alkyl) may be accomplished using standard alkylation methods well known in art. Reduction of compounds wherein B is -CO(C₁-C₃ alkyl) using standard methods well known in the art will afford the corresponding compounds wherein R³ is one of various (C₁-C₄ alkyl) derivatives.

Compounds of the formula IV-a wherein A is CR⁷ and G, J, and K are carbon may be prepared by heating compounds of formula VI-a with an appropriate compound of the formula R-³C(O)CR⁷COO(C₁-C₄ alkyl) under acid or Lewis acid conditions with or without a solvent, as shown in Scheme 1. Examples of such reaction conditions are: a) heating in polyphosphoric acid; b) heating in toluene, benzene or xylene in the presence of acid catalyst (such as p-TsOH, sulfuric acid or HCl(g)) using a Dean-Stark trap; or c) heating in an appropriate solvent such as dichloroethane, diphenylether (Ph₂O) or Dowtherm A in the presence of a Lewis acid such as SnCl₄, ZnCl₂/HCl or AlCl₃.

Compounds of formula VI-a may be prepared using methods described in the scientific literature. (See Gazz. Chim. Ital.; 111, p167-172(1981); Chem. Pharm. Bull.; 24, 3001-3010 (1976); J. Org. Chem., 38, 1777-1780 (1973); Chem. Abstr. 68, 68982f (1968); Aust. J. Chem.; 22, 563-572(1969); J. Chem. Soc. Perkin Trans.2, p1954, p1957 (1972); J. Heterocycl. Chem.; FR, 19, 443-445(1982); J. Heterocycl. Chem., 22, 1496-1502 (1985); Tetrahedron, 47, 4639-4644 (1991); J. Heterocycl. Chem., 28, 2053-2055 (1991); J. Heterocycl. Chem., 29, 251-153 (1992).

Compounds of formula V-a and IV-a', wherein R is C₁-C₄alkyl can be prepared by heating compounds of formula VI wherein W is -CN and -COO(C₁-C₄ alkyl), respectively, with an appropriate R³C(O)CH₂COO(C₁-C₄ alkyl) in the presence of a Lewis acid such as SnCl₄, AlCl₃, TiCl₃ or ZnCl₂, in a inert solvent such as dichloroethane, at about the reflux temperature, as shown in Scheme 2. Base hydrolysis of a compound of the formula V-a or IV-a' with sodium hydroxide in H₂O/(C₁-C₄ alcohol) at reflux, or with lithium hydroxide in water/THF or water/dioxane at temperature from about room temperature to about the reflux temperature, followed by decarboxylation by heating in a oil bath at a temperature from about 140-180°C will give a compound of formula V-b or IV-b, respectively. Compounds of the formulas V-a and IV-a' may be converted into compounds of the formula II-a, and compounds of the formulas V-b and IV-b may be converted into compounds of the formula II-b by the procedures described above. The conversion of compounds of the formula II-a into those of the formula II-c can be accomplished using procedures analogous to those described above for converting compounds wherein R³ is -COO(C₁-C₄ alkyl) into those wherein R³ is C₁-C₄ alkyl.

Compounds of formula VI may be prepared using methods described in the literature. (See Liebigs Ann Chem., 1534-1546,1979; Gazz. Chim. Ital., 97, 25-33,1967; Gazz. Chim. Ital., 120, 725-730, 1990; Eur. J. Med. Chem. Chim. Ther., 26, 143-158, 1991; J. Heterocycl. Che., Fr. 19, 443-445, 1982; J. Heterocycl. Chem., 22, 1496-1502, 1985; J. Heterocycl. Chem., 31, 305-312, 1994; J. Heterocycl. Chem., 24, 243-245, 1987; J. Org. Chem. 57, 3713-3716, 1992; Liebigs Ann Chem., 1702-1710, 1984; Chem. Pharm. Bull., 34, 701-702,1986; Pharmazie, 48, 849-853, 1993; Bull. Soc. Chim. Belg., 103, 181-184, 1994; and Indian. J. Chem. Sect. B, 33, 436-440, 1994).

Compounds of formula IV-c wherein A is N and G, J, and K are carbon may be prepared, as shown in Scheme 3, by reacting compounds of formula VI with (R³CO)₂O, R³COOH or R³CO(OC₁-C₂ alkyl)₃, in acetic acid or an appropriate inert organic solvent such as toluene, dioxane, acetonitrile, methylene chloride or chloroform, at a temperature from about 25°C to about 150°C, preferably at about the reflux temperature, followed by heating in 85% phosphoric acid or an aqueous acid such as acetic acid, hydrochloric acid or sulfuric acid, preferably in 50-85% phosphoric acid. Compounds of formula V-b may be prepared, as shown in Scheme 3, by heating the corresponding compounds of formula VI with excess of the appropriate compound of the formula R³CONH₂.

Compounds of formula IV-d may be prepared, as shown in Scheme 4, by reacting compounds of formula IX with an appropriate reagent having the formula R³C(O)CHR⁷COO(C₁-C₄alkyl) in an R³COOH solvent at temperature from about 60°C to about 180°C, preferably at about the reflux temperature.

Compounds of formula I-N may be prepared, as shown in Scheme 5, by reacting the corresponding compounds of formula X with a (C₁-C₇ alkyl)nitrite, with or without CuBr₂, CuCl₂, or Cul₂, in an appropriate inert solvent such as acetonitrile, acetone, methylene chloride, chloroform, benzene or toluene, preferably acetonitrile at a temperature from about 25°C to about 150°C, preferably from about 60°C to 100°C.

Compounds of formula I-L and I-M may be prepared, as shown in Scheme 6, by reacting compounds of formula XI wherein X is S or O with a compound of the formula R⁴CHO or R⁴CH(OC₁-C₂ alkyl)₂ and an acid catalyst such as p-TsOH, HCl, HBr, H₂SO₄, or HCl, in toluene, xylene or benzene, preferably toluene, with from one to ten equivalents of water, at temperature from about 70°C to about 160°C, using a Dean-Stark trap or in the presence of anhydrous sodium sulfate.

Compounds of formula I-K and I-U may be prepared by reacting the corresponding compounds of formula XI with triphosgene and thiophosgene, respectively, and a base such as triethylamine or pyridine in an appropriate inert solvent such as methylene chloride, THF, dioxane, ether, benzene or chloroform, preferably methylene chloride or dry THF, at temperature from about 0°C to about 25°C.

Compounds of formula I-V, I-W, and I-X may be prepared, as illustrated in Scheme 7, starting from compounds of formula XII. Compounds of formula XIII may be prepared by reacting the corresponding compounds of formula XII with hydroxylamine in acidic (e.g., in trifluoroacetic acid) or basic (e.g., in NaOAc or NaOH and hydroxylamine hydrochloride in a C₁-C₄ alcohol/water mixture) conditions at temperatures from about 25°C to about 150°C, preferably at about the reflux temperature. Compounds of formula XIV can be prepared by heating the corresponding compounds of formula XIII in acetic anhydride, trifluoroacetic anhydride or Tf₂O, with or without a solvent such as acetic acid or methylene chloride, In the presence of an appropriate amine base such as triethylamine or pyridine. Compounds of formula I-V, I-W and I-X may be prepared by heating the corresponding compounds of formula XIV and pyridine in an inert solvent such as DMF, DMSO, NMP, sulfolane or acetamide at a temperature from about 80°C to about 180°C.

Compounds of formula I-G may be prepared, as illustrated in Scheme 8, by reducing the corresponding compounds of formula XVII. This reduction can be performed using standard methods known in literature for reduction of a nitro group to an amino group. Such methods include hydrogenation or reduction by iron in acetic acid. Cyclization may occur upon reduction or heating in an appropriate solvent such as a C₁-C₄ alcohol, acetonitrile, toluene, THF, methylene chloride or acetic acid.

The conversion of compounds of formula XVI into those of formula XVII can be accomplished using methods analogous to those described above for the conversion of compounds of formula V wherein W is cyano into compounds of formula I wherein B is a group having a carbon atom directly attached to the bicyclic ring. The best method for conversion of a cyano group to a -COOH group is acid hydrolysis, for example, heating the cyano compound in 50-85% phosphoric acid or 50-90% acetic acid, preferably phosphoric acid. The best method for converting a cyano group into a -CO(C₁-C₄ alkyl) is reacting the cyano compound with a Grignard reagent at a temperature from about 0°C to about 25°C in ether, THF or dioxane. The best method for converting a cyano group into a -CHO group is a diisobutylaluminum hydride reduction in THF, dioxane, or ether at a temperature from about -78°C to about 25°C, preferably from about -78°C to about -40°C.

Compounds of the formula XVI may be prepared by reacting compounds of formula XV, wherein Hal is chloro, bromo or iodo, with a sodium, potassium, or lithium salt of R⁵CH₂CN in an appropriate inert solvent such as toluene, benzene, a C₁-C₅ alcohol, THF, DMSO, dioxane, or pyridine, with or without a Pd(II) or Pd(0) catalyst, at a temperature from about -78°C to about 130°C.

Alternatively, compounds of formula XVII may be prepared by subjecting compounds of the formula XV to halogen-metal exchange (e.g., using an organo lithium agent such as tBuLi, s-BuLi or BuLi at -78°C in ether, THF, or dioxane), followed by quenching with an electorphile such as R⁵CHO, to give compounds of formula XVIII. Compounds of formula XIX-a may be prepared by reacting the corresponding compounds of formula XVIII with thionyl chloride, followed by reduction. Reacting compounds of formula XIX-a with a base (such as organolithium agent (e.g., lithium diisopropylamide or BuLi), followed by quenching with an electrophile (such as carbon dioxide) will yield the corresponding compounds of formula XIX-b wherein R²¹ is -COOH. Compounds of formula XIX-c wherein R²¹ is -C(OH)R⁴ may be prepared in a similar fashion by quenching the appropriate compound of formula XIX-a with an electrophile of the formula C₁-C₃ alkyl-CHO. Compounds of formula XVII can be prepared by reacting compounds of formula XIX-c with PCC (pyridinium chlorochromate) using standard PCC oxidation methods that are well known in the art.

Compounds of formula II-d, II-e and II-f, wherein T is chloro, bromo or iodo and D, E, R³, R⁵ and R⁷ are as defined to those in formula I may be prepared, as shown in Scheme 9, by methods analogous to those described in Scheme 2. Alternatively, Compounds of formula II-d to II-f may be prepared using procedures analogous to those described in the literature (See: Pharm. Bull, 5, 229-231, 1957; Monatsh Chem, 100, 671-678, 1969; J. Heterocycl. Chem., 3, 218-220, 1966, Chem. Pharm. Bull,. 23, 2891-2895, 1975; J. Heterocycl. Chem., 8, 1-6, 1971; Justus Liebigs Ann. Chem, 735, 35-44, 1970; Tetrahedron Lett, 1479, 1968; and Chem. Abstr., 1939, 4988.)

The acid addition salts of compounds of the formula can be prepared in a conventional manner by treating a solution or suspension of the corresponding free base with one chemical equivalent of a pharmaceutically acceptable acid. Conventional concentration or crystallization techniques can be employed to isolate the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzene sulfonic, p-toluenesulfonic, and related acids.

The compounds of formula I and their pharmaceutically acceptable salts (hereinafter referred to, collectively, as "the active compounds of this invention") may be administered alone or in combination with pharmaceutically acceptable carriers, in either single or multiple doses. Suitable pharmaceutical carriers include inert solid diluents or fillers, sterile aqueous solutions, oils (e.g., sesame oil, peanut oil) and various organic solvents. The pharmaceutical compositions formed by combining the compounds of formula I and pharmaceutically acceptable carriers can then be readily administered in a variety of dosage forms such as tablets, oil gel, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions containing an active compound of this invention or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

The effective dosages for the active compounds of this invention will depend on the intended route of administration and factors such as the age and weight of the patient, as generally known to a physician. The dosages will also depend on the particular illness to be treated. For instance, the daily dosage for stress-induced illnesses, inflammatory disorders, Alzheimer's disease, gastro-intestinal diseases, anorexia nervosa, hemorrhagic stress and drug and alcohol withdrawal symptoms will generally range from about 0.1 to about 50 mg/kg body weight of the patient to be treated.

Methods that may be used to determine the CRF antagonist activity of the active compounds of this invention and their pharmaceutically acceptable salts are described in Endocrinology, 116, 1653-1659 (1985) and Peptides, 10, 179-188 (1985). The binding activities for compounds of the formula I, expressed as IC₅₀ values, generally range from about 0.5 nanomolar to about 10 micromolar. Methods that can be used to determine the CRF binding protein inhibiting activity of compounds of the formula I are described in Brain Research, (1997), 745 (1,2), 248-255.

The present invention is illustrated by the following examples. Melting points are uncorrected. Proton nuclear magnetic resonance spectra (¹H NMR) and C¹³ nuclear magnetic resonance spectra (C¹³ NMR) were measured for solutions in deuterochloroform (CDCl₃) and peak positions are expressed in parts per million (ppm) downfield from tetramethylsilane (TMS). The peak shapes are denoted as follows: s, singlet; d, doublet; t, triplet; q, quartet; m, multiplet; b, broad.

The following abbreviations are used in the Examples: Ph=phenyl; iPr=isopropyl.

### EXAMPLE 1

### 7-(1-Ethyl-propoxy)-5-methyl-3-(2,4,6-trimethyl-phenyl)-pyrazolo[1,5-a]pyrimidine

A solution of 3-pentanol (140 mg, 1.5 mmol) in 1 ml of dry THF was treated with 60% sodium hydride in oil (28 mg, 0.7 mmol) and stirred at room temperature for 10 min. A solution of 7-chloro-5-methyl-3-(2,4,6-trimethyl-phenyl)-pyrazolo[1,5-a]pyrimidine (75 mg, 0.262 mmol) in 1 ml of dry THF was added and the resulting mixture was heated at reflux for 5 hours. The mixture was quenched with water and extracted with ethyl acetate. The organic layer was washed with brine, dried and concentrated to give the crude material. The residue was purified through silica gel column chromatography using 3:7 chcloroform:hexane as eluent to give 75mg (88%) of the title compound. ¹H NMR (CDCl₃) δ 7.97(s,1H), 6.97(s,2H), 6.03(s,1H), 4.56 (m,1H), 2.53(s,3H), 2.32(s,3H), 2.13(s,6H), 2.10(m,4H), 1.09(t,6H) ppm.

### EXAMPLE 2

### [2,5-Dimethyl-3-(2,4,6-trimethyl-phenyl)-pyrazolo[1,5-a]pyrimidin-7-yl]-(1-ethylpropyl)-amine

A mixture of 7-Chloro-2,5-dimethyl-3-(2,4,6-trimethyl-phenyl)-pyrazolo[1,5-a] pyrimidine (60 mg, 0.2 mmol) and 1-ethylpropylamine (4 ml) in 1ml of N-methylpyrrolidinone was heated at 125°C oil bath for 15 hrs. The mixture was quenched with water and extracted with ethyl acetate. The organic layer was dried and concentrated and purified through silica gel column chromatography using 20% ethyl acetate in hexane as eluent to give 35 mg of the title compound as a yellow solid. ¹H NMR (CDCl₃) δ 6.96(s,2H), 6.00(d,1H), 5.77(s,1H), 3.47 (m,1H), 2.43(s,3H), 2.32(s,3H), 2.22(s,3H), 2.05(s,6H), 1.5-1.9(m,4H), 1.04(t,6H) ppm.

### EXAMPLE 3

### (1-Ethyl-propyl)-[5-methyl-3-(2,4,6-trimethyl-phenyl)-pyrazolo[1,5-a]pyrimidin-7-yl]-amine

A mixture of 7-chloro-5-methyl-3-(2,4,6-trimethyl-phenyl)-pyrazolo[1,5-a] pyrimidine (190 mg, 0.63 mmol) and 1-ethylpropylamine (4 ml) In 1ml of N-methylpyrrolidinone was heated at 125°C oil bath for 15 hours. The mixture was quenched with water and extracted with ethyl acetate. The organic layer was dried and concentrated and purified through silica gel column chromatography using chloroform to 2% methanol in chloroform as eluent to give 195 mg (87%) of the title compound as a green solid. ¹H NMR (CDCl₃) δ 7.87(s,1H), 6.97(s,2H), 6.12(d,1H), 6.85(s,1H), 3.52(m,1H), 2.48(s,3H), 2.09(s,3H), 2.16(s,6H), 1.6-1.9(m,4H), 1.05(t,6H)ppm.

### EXAMPLE 4

### 7-(1-Ethyl-propoxy)-2,5-dimethyl-3-(2,4,6-trimethyl-phenyl)-pyrazolo [1,5-a]pyrimidine

To a suspension of 60% Sodium hydride in oil (160 mg) in 4 ml of DMSO was added 3-pentanol (853 mg), and then 7-chloro-2,5-dimethyl-3-(2,4,6-trimethyl-phenyl) -pyrazolo[1,5-a]pyrimidine (580 mg) at room temperature. The mixture was heated at 88°C overnight. The mixture was quenched with water and extracted with ethyl acetate. The organic layer was dried and concentrated and purified through silica gel column chromatography using 10% hexane in chloroform as eluent to give the title compound as an orange oil. ¹H NMR (CDCl₃) δ 6.96(s,2H), 5.95(s,1H), 4.52(m,1H), 2.48(s,3H), 2.33(s,3H), 2.27(s,3H), 2.03(s,6H), 1.75-2.00(m,4H), 1.08(t,6H)ppm.

### EXAMPLE 5

### [2,5-Dimethyl-3-(2,4,6-trimethyl-phenyl)-pyrazolo[1,5-a]pyrimidin-7-yl]-ethyl-propyl-amine

A mixture of 7-chloro-5-methyl-3-(2,4,6-trimethyl-phenyl)-pyrazolo[1,5-a] pyrimidine (200 mg, 0.66 mmol) and N-propylethylamine (2 ml) in 1 ml of N-methylpyrrolidinone was heated at 135°C oil bath for 4 hours. The mixture was quenched with water and extracted with ethyl acetate. The organic layer was dried and concentrated and purified through silica gel column chromatography using hexane to 10% ethyl acetate in hexane as eluent to give 150 mg of the title compound as a clear green oil. ¹H NMR (CDCl₃) δ 6.95(s,2H), 5.80(s,1H), 3.85(q,2H), 3.67(dd,2H), 2.41 (s,3H), 2.32(s,3H), 2.21 (s,3H), 2.03(s,6H), 1.76(m,2H), 1.29(t,3H), 0.98(t,3H) ppm.

### EXAMPLE 6

### [6-Bromo-5-bromomethyl-3-(2,4,6-trimethyl-phenyl)-3H-[1,2,3]triazolo[4,5-b] pyridin-7-yl]-(1-ethyl-propyl)-amine

To a solution of butyl nitrite (119 mg, 1.15 mmol) and CuBr2 (205 mg, 0.919 mmol) in 16 ml of acetonitrile was added N4-(1-ethyl-propyl)-6-methyl-N2-(2,4,6-trimethyl-phenyl)-pyridine-2,3,4-triamine (250 mg, 0.766 mmol). The resulting mixture was heated at 65°C for 1.5 hrs. The mixture was cooled to room temperature and 2N HCl (16 ml) was added. The mixture was neutralized with 2N NaOH and extracted with ethyl acetate. The organic layer was washed with brine, dried and concentrated to give a brown oil. The oil residue was-purified through silica gel column chromatography using 1:1 hexane :ethyl acetate as eluent to give 61mg of [6-bromo-5-bromomethyl-3-(2,4,6-trimethyl-phenyl)-3H-[1,2,3]triazolo[4,5-b] pyridin-7-yl]-(1-ethyl-propyl)-amine as white crystals. Mp 123-125°C. ¹H NMR (CDCl₃) δ 7.06(s,2H), 5.53(d,1H), 5.22(m,1H), 4.67(s,2H), 2.39(s,3H), 1.96(s,6H), 1.6-1.9(m,4H), 1.06(t,6H)ppm and 103 mg of [6-bromo-5-methyl-3-(2,4,6-trimethyl-phenyl)-3H-[1,2,3]triazolo[4,5-b]pyridin-7-yl]-(1-ethyl-propyl)-amine as white solid. Mp 115-117°C. ¹H NMR (CDCl₃) δ 7.04(s,2H), 5.36(d,1H), 05.21(m,1H), 2.64(s,3H), 2.38(s,3H), 1.95(s,6H), 1.6-1.9(m,4H), 1.05(t,6H)ppm.

### EXAMPLE 7

### (1-Ethyl-propyl)-[5-methyl-3-(2,4,6-trimethyl-phenyl)-3H-[1,2,3]triazolo[4.5-b] pyridin-7-yl]-amine

A mixture of butyl nitrite (119 mg, 1.15 mmol) and N4-(1-ethyl-propyl)-6-methyl-N2-(2,4,6-trimethyl-phenyl)-pyridine-2,3,4-triamine (250 mg, 0.766 mmol) in anhydrous acetonitrile (16 ml) was heated at 65°C for 2 hours. The mixture was cooled to room temperature and 2N HCl (16 ml) was added. The mixture was neutralized with 2N NaOH and extracted with ethyl acetate. The organic layer was washed with brine, dried and concentrated to give 250 mg of the crude product as a brown oil. The oil residue was purified through silica gel column using chloroform as eluent to give 201 mg of the title compound as golden yellow solid. Mp 131-133°C. ¹H NMR (CDCl₃) δ 7.022(s,2H), 6.20(s,1H), 5.44(d,1H), 3.65(m,1H), 2.50(s,3H), 2.36(s,3H), 1.96(s,6H), 1.5-1.8(m,4H), 1.03(t,6H)ppm.

### EXAMPLE 8

### [6-Bromo-5-methyl-3-(2,4,6-trimethyl-phenyl)-3H-[1,2,3]triazolo[4,6-b]pyridin-7-yl]-(1-ethyl-propyl)-methyl-amine

To a solution of [6-bromo-5-methyl-3-(2,4,6-trimethyl-phenyl)-3H-[1,2,3]triazolo[4,5-b]pyridin-7-yl]-(1-ethyl-propyl)-amine (50 mg, 0.12 mmol) In dry THF)1.5 ml) was added 2.5 M n-BuLi in hexane (0.14 ml) at -78°C. After stirring at -78°C for 10 min, 0.5 ml of methyl iodide was added at that temperature then allowed to warmed to room temperature and stirred for 15 min for 2 hours. The mixture was quenched with water and extracted with ethyl acetate. The organic layer was washed with brine, dried and concentrated to give 46 mg of brown oil. The residue was purified through silica gel coliumn chromatography using 1:1 chloroform: hexane as eluent to give the title compound as a golden oil. ¹H NMR (CDCl₃) δ 7.04(s,2H), 4.35(m,1H), 3.32(s,3H), 2.70(s,3H), 2.38(s,3H), 1.94(s,6H), 1.7-2.0(m,4H), 1.01(t,6H)ppm.

### EXAMPLE 9

### 7-(1-Ethyl-propoxy)-5-methyl-3-(2,4,6-trimethyl-phenyl)-3H-[1,2,3]triazolo[4,5-b]pyridine

A mixture of 4-(1-Ethyl-propoxy)-6-methyl-N2-(2,4,6-trimethyl-phenyl)-pyridine-2,3-diamine (50 mg, 0.153 mmol) and butyl nitrite (24 mg, 0.229 mmol) in 4 ml acetonitrile was heated at 65°C for 2 hours. An additional 0.13 ml of butyl nitrite was added and the resulting mixture was heated at 65°C for 2 hours. The mixture was quenched with water and extracted with ethyl acetate. The organic layer was dried and concentrated to give 58 mg of brown oil. The oil was purified through silica gel column chromatography using 5% ethyl acetate in hexane as eluent to give 46 mg (88%) of the title compound as a light yellow oil. ¹H NMR (CDCl₃) δ 7.04(s,2H), 6.60(s,1H), 5.26(m,1H), 2.57(s,3H), 2.38(s,3H), 1.94(s,6H), 1.8-2.0(m, 4H), 1.07(t,6H) ppm.

### EXAMPLE 10

### 4-(1-Ethyl-propoxy)-2,5-dimethyl-7-(2,4,6-trimethyl-phenyl)-5H-pyrrolo[3,2-d]pyrimidine

To a solution of 3-pentanol (0.09 ml, 0.883 mmol) in dry THF was added 60% NaH in oil (20 mg, 0.500 mmol) and stirred for 5 min. A solution of 4-chloro-2,5-dimethyl-7-(2,4,6-trimethyl-phenyl)-5H-pyrrolo[3,2-d]pyrimidine (50 mg, 0.166 mmol) in dry THF was added to the reaction mixture and the resulting mixture was heated at reflux for 2 hr. The mixture was quenched with water, extracted with ethyl acetate. The organic layer was washed with brine, separated, dried and concentrated to dryness to give the title compound as a white solid. ¹H NMR (CDCl₃) δ 6.92(s,3H), 5.43(m,1H), 4.02(s,3H), 2.56(s,3H), 2.29(s,3H), 2.08(s,6H), 1.80(m,4H), 0.99(t,6H).

The title compounds of Examples 11-14 were prepared by the method analogous to that described in Example 10 starting from of 4-chloro-2-mothyl-6-substituted-7-(substituted-phenyl)-5H-pyrrolo[3,2-d]pyrimidine or 7-chloro-5-methyl-1-substituted-3-(substituted-phenyl)-1H-pyrrolo[3,2-b]pyridine and an appropriate alcohol or thiol and a base.

### EXAMPLE 11

### (+)-2,5-Dimethyl-4-(tetrahydro-furan-3-yloxy)-7-(2,4,6-trimethyl-phenyl)-5-pyrrolo [3,2-d]pyrimidine,

¹H NMR (CDCl₃) δ 6.95(s,1H), 6.92(s,2H), 5.88(m,1H), 3.9-4.08(m,4H), 4.01 (s,3H), 2.56(s,3H), 2.29(s,3H), 2.2-2.4(m,2H), 2.07(s,6H) ppm.

### EXAMPLE 12

### 2,5-Dimethyl-4-(S)-(tetrahydro-furan-3-yloxy)-7-(2,4,6-trimethyl-phenyl)-5H-pyrrolo[3,2-d]pyrimidine.

¹H NMR (CDCl₃) δ 6.95(s,1H), 6.92(s,2H), 5.88(m,1H), 3.9-4.08(m,4H), 4.01 (s,3H), 2.56(s,3H), 2.29(s,3H), 2.2-2.4(m,2H), 2.07(s,6H) ppm.

### EXAMPLE 13

### 2,5-Dimethyl-4-(1-propyl-butoxy)-7-(2,4,6-trimethyl-phenyl)-5H-pyrrolo[3,2-d]pyrimidine.

¹H NMR (CDCl₃) δ 6.93(s,2H), 6.92(s,1H), 6.58(m,1H), 4.02(s,3H), 2.56(s,3H), 2.29(s,3H), 2.09(s,6H), 1.6-1.8(m,4H), 1.4-1.6(m,4H), 0.96(t,6H)ppm.

### EXAMPLE 14

### 4-sec-Butylsulfanyl-2,5-dimethyl-7-(2,4,6-trimethyl-phenyl)-5H-pyrrolo[3,2-d]pyrimidine.

¹H NMR (CDCl₃) δ 6.97(s,1H), 6.94(s,2H), 4.34(m,1H), 4.13(s,3H), 2.63(s,3H), 2.30(s,3H), 2.07(s,6H), 1.7-1.9(m,2H), 1.48(d,3H), 1.09(t,3H) ppm.

The title compounds of Examples 15-18 were prepared using the following procedure.

### PROCEDURE FOR EXAMPLES 15 - 18

A mixture of 4-chloro-2-methyl-5-substituted-7-(substituted-phenyl)-5H-pyrrolo[3,2-d]pyrimidine or 7-bromo-1,5-dimethyl-3-(2,4,6-trimethyl-phenyl)-1H-pyrrolo[3,2-b]pyridine-6-carboxylic acid methyl ester (1 mmol) and an appropriate amine in DMSO (2 ml) was heated in 130°C oil bath until all the starting material was consumed. The mixture was quenched with water and extracted with ethyl acetate. The organic layer was dried and concentrated to dryness to give the corresponding 4-alkylamino-2-methyl-5-substituted-7-(substituted-phenyl)-5H-pyrrolo[3,2-d]pyrimidineor 7-alkylamino-1,5-dimethyl-3-(2,4,6-trimethyl-phenyl)-1H-pyrrolo[3,2-b]pyridine-6-carboxylic acid methyl ester derivative. Silica gel column chromatography may be used for purification.

### EXAMPLE 15

### [2,5-Dimethyl-7-(2,4,6-trimethyl-phenyl)-5H-pyrrolo[3,2-d]pyrimidin-4-yl]-(1-ethyl-propyl)-amine

¹H NMR (CDCl₃) δ 6.91 (s,2H), 6.76(s,1H), 4.61 (d,1H,NH), 4.33(m,1H), 4.04(s,3H), 2.49(s,3H), 2.28(s,3H), 2.09(s,6H), 1.72(m,2H), 1.60(m,2H), 0.98(t,6H)ppm.

### EXAMPLE 16

### Butyl-[2,5-dimethyl-7-(2,4,6-trimethyl-phenyl)-5H-pyrrolo[3,2-d]pyrimidin-4-yl]-ethyl-amine

¹H NMR (CDCl₃) δ 6.96(s,1H), 6.92(s,2H), 3.93 (s,3H), 3.44(q,2H), 3.40(m,2H), 2.57(s,3H), 2.29(s,3H), 2.09(s,6H), 1.57(m,2H), 1.30(m,2H), 1.14(t,3H), 0.88(t,3H)ppm.

### EXAMPLE 17

### 2,5-Dimethyl-4-thiazolidin-3-yl-7-(2,4,6-trimethyl-phenyl)-5H-pyrrolo[3,2-d]pyrimidine

¹H NMR (CDCl₃) δ 7.03(s,1H), 6.92(s,2H), 4.78(s,2H), 4.02(s,3H), 3.96(m,2H), 3.18 (m, 2H), 2.56(s,3H), 2.29(,3H), 2.06 (s,6H) ppm.

### EXAMPLE 18

### 7-(1-Ethyl-propylamino)-1,5-dimethyl-3-(2,4,6-trimethyl-phenyl)-1H-pyrrolo[3,2-b] pyridine-6-carboxylic acid methyl ester

¹H NMR (CDCl₃) δ 6.98(s,1H), 6.93(s,2H), 5.40(d,1H), 3.97(s,3H), 3.91(s,3H), 3.35(m,1H), 2.57(s,3H), 2.30(s,3H), 2.09(s,6H), 1.52(m,4H), 0.87(t,6H) ppm.

### EXAMPLE 19

### 7-(1-Ethyl-propylamino)-1,5-dimethyl-3-(2,4,6-trimethyl-phenyl)-1H-pyrrolo[3,2-b]pyridine-6-carboxylic acid

A mixture of 7-(1-ethyl-propylamino)-1,5-dimethyl-3-(2,4,6-trimethyl-phenyl)-1H-pyrrolo[3,2-b]pyridine-6-carboxylic acid methyl ester and NaOH in a 1:1 mixture of MeOH/water was heated at reflux over night. The resulting mixture was acidified with 2N HCl to a pH of 4-5, and extracted with chloroform. The organic layer was dried and concentrated to give the title compound.

¹H NMR (CDCl₃) δ 7.02(s,1H), 6.82(s,2H), 3.98(s,3H), 3.78(m,1H), 2.59(s,3H), 2.07(s,3H), 2.00(s,6H), 1.64(m,4H), 0.90(t,6H) ppm.

### EXAMPLE 20

### [1,5-Dimethyl-3-(2,4,6-trimethyl-phenyl)-1H-pyrrolo[3,2-b]pyridin-7-yl]-(1-ethyl-propyl)-amine

A mixture of 7-(1-ethyl-propylamino)-1,5-dimethyl-3-(2,4,6-trimethyl-phenyl)-1H-pyrrolo[3,2-b]pyridine-6-carboxylic acid was heated in 150-160°C oil bath until all the starting material was consumed. ¹H NMR (CDCl₃) δ 6.94(s,2H), 6.87(s,1H), 6.15(s,1H), 6.10(d,1H), 4.24(s,3H), 3.50(m,1H), 2.64(s,3H), 2.30(s,3H), 2.05(s,6H), 1.77(m,4H), 1.02(t,6H)ppm.

### PREPARATION A

### 4-(2,4,6-Trimethyl-phenyl)-2H-pyrazol-3-ylamine

A mixture of 3-oxo-2-(2,4,6-trimethyl-phenyl)-propionitrile (2.300 g, 12.3 mmol), hydrazine hydrate (0.93 g) and glacial acetic acid (1.55 ml) in 20 ml benzene was heated at reflux for 4.5 hours. Reaction mixture was cooled to room temperature and 50 ml of 18.5% HCl in water was added. The benzene layer was separated and reextracted with 18.6% HCl. The aqueous layer were combined and neutralized with ammonium hydroxide and stirred at rt. overnight. Precipitate formed and was filtered to yield the title compound (0.256 g) as a yellow solid. The benzene layer was concentrated and purified through silica gel column using 5% methanol in chloroform as eluent to give an additional 1.450 g of the title compound; ¹H NMR (CDCl₃) δ 7.24(s,1H), 6.95(s,2H), 4.75(brs,2H), 2.32(s,3H),2.13(s,6H) ppm.

### PREPARATION B

### 5-Methyl-4-(2,4,6-trimethyl-phenyl)-2H-pyrazol-3-ylamine

The title compound was prepared as white solid by the method analogous to that described in preparation A starting from 3-oxo-2-(2,4,6-trimethyl-phenyl)-butyronitrile. ¹H NMR (CDCl₃) δ 7.7(brs,1H). 6.96(s,2H), 2.32(s,3H), 2.13(s,3H), 2.06(s,6H) ppm.

### PREPARATION C

### 2,5-Dimethyl-3-(2,4,6-trimethyl-phenyl)-4H-pyrazolo[1,5-a]pyrimidin-7-one

Methyl acetoacetate (0.38 ml) was added to a solution of 5-methyl-4-(2,4,6-trimethyl-phenyl)-2H-pyrazol-3-ylamine (641 mg, 2.98 mmol) in 4 ml of acetic acid. The reaction mixture was heated at reflux for overnight. The mixture was concentrated to dryness and the residue was purified through silica gel column chromatography using 5% methanol in chloroform as eluent to give 560 mg (65.5%) of the title compound as a white solid; ¹H NMR (DMSO-d₆) δ 11.7(s,1H), 6.98(s,2H), 5.52(s,1H), 2.29(s,3H), 2.20(s,3H), 1.97(s,3H), 1.95(s,6H) ppm.

### PREPARATION D

### 5-Methyl-3-(2,4,6-trimethyl-phenyl)-4H-pyrazolo[1,5-a]pyrimidin-7-one

Methyl acetoacetate (0.7 ml) was added to a solution of 4-(2,4,6-Trimethyl-phenyl)-2H-pyrazol-3-ylamine (1.120 g, 5.57 mmol) in 5 ml of acetic acid and the resulting mixture was heated at reflux for two days. Reaction mixture cooled and a white solid formed. Ethanol (6 ml) was added and stirred at room temperature overnight, filtered to give white solid which was recrystallized from ethanol to give 673 mg(45.2%) of the title compound as white crystals. ¹H NMR (DMSO-d₆) δ 11.9(s,1H), 7.7(s,1H), 6.95(s,2H), 5.55(s,1H), 2.26(s,3H), 2.20(s,3H), 2.0(s,6H)ppm.

### PREPARATION E

### 7-Chloro-5-methyl-3-(2,4,6-trimethyl-phenyl)-pyrazolo[1,5-a]pyrimidine

A suspension of 5-methyl-3-(2,4,6-trimethyl-phenyl)4H-pyrazolo[1,5-a]pyrimidin -7-one (590 mg, 2.2 mmol) in 9 ml of POCl₃ was treated with diethylaniline (0.7 ml) and the resulting mixture was stirred at reflux for 15 hours. The reaction mixture was concentrated to dryness. The residue was treated with ice water and stirred for 20 min, then extracted with chloroform. The organic layer was dried and concentrated to yield an orange oil which crystallized upon standing. The material was purified through silica gel column chromatography using chloroform as eluent to give 590 mg (94%) of the title compound as a yellow solid; ¹H NMR (CDCl₃) δ 8.08(s,1H), 6.98(s,2H), 6.86(s,1H), 2.56(s,3H), 2.33(s,3H), 2.09(s,6H) ppm.

### PREPARATION F

### 7-Chloro-2,5-dimethyl-3-(2,4,6-trimethyl-phenyl)-pyrazolo[1,5-a]pyrimidine

The title compound was prepared as an oil by the method analogous to that described in preparation E starting from 2,5-dimethyl-3-(2,4,6-trimethyl-phenyl)-4H-pyrazolo[1,5-a]pyrimidin-7-one. ¹H NMR (CDCl₃) δ 6.98(s,2H), 6.77(s,1H), 2.51(s,3H), 2.33(s,3H), 2.31 (s,3H), 1.99(s,6H) ppm.

### PREPARATION G

### 2,5-Dimethyl-7-(2,4,6-trimethyl-phenyl)-5H-pyrrolo[3,2-d]pyrimidin-4-ol

A mixture of 3-amino-1-methyl-4-(2,4,6-trimethylphenyl)-1H-pyrrole-2-carbonitrile (0.4 mmol) and acetic anhydride (0.043 ml) in acetic acid (0.01 ml) was heated at reflux untill all the starting material was consumed. The reaction mixture was concentrated to dryness. The residue was quenched with water and extracted with ethyl acetate. The organic extracts was washed with brine and concentrated to dryness. The residue was suspended in 0.5 ml of 85% phosphoric acid and heated at 130°C for 1 hour. The mixture was cooled and poured into ice-water, stirred until solid formed. The solid was filtered to give 2,5-dimethyl-7-(2,4,6-trimethylphenyl)-5H-pyrrolo[3,2-d]pyrimidin-4-ol. The alternative way for the work up is via extraction technique. The Ice water was extracted with chloroform. The organic layer was dried and concentrated to dryness to give the desired product.
¹H NMR δ (CDCl₃) 6.92(s,2H), 6.88(s,1H), 4.14(s,3H), 2.43(s,3H), 2.29(s,3H), 2.09(s,6H).

The following compounds can be prepared in a similar manner:
2,5-Dimethyl-7-(2,6-dimethyl-4-chloro-phenyl)-5H-pyrrolo[3,2-d]pyrimidin-4-ol;
2,5-Dimethyl-7-(2,6-dimethyl-4-bromo-phenyl)-5H-pyrrolo[3,2-d]pyrimidin-4-ol;
2-Methyl-7-(2,4,6-trimethyl-phenyl)-5H-pyrrolo[3,2-d]pyrimidin-4-ol;
2-Methyl-7-(2,6-dimethyl-4-chloro-phenyl)-5H-pyrrolo[3,2-d]pyrimidin-4-ol;
2-Methyl-7-(2,6-drimethyl-4-bromo-phenyl)-5H-pyrrolo[3,2-d]pyrimidin-4-ol; and
2-Methyl-7-(2,4,6-trimethyl-phenyl)-5H-pyrrolo[3,2-d]pyrimidin-4-ol;

### PREPARATION H

### 4-Chloro-2,5-dimethyl-7-(2,4,6-trimethyl-phenyl)-5H-pyrrolo[3,2-d]pyrimidine

A mixture of 2, 5-dimethyl-7-(2,4,6-trimethylphenyl)-5H-pyrrolo[3,2-d]pyrimidin-4-ol (1 mmol) in POCl₃ (1.3 ml) was heated at reflux until all the starting material were consumed (about 1-3 hours). The mixture was concentrated to dryness. The residue was porued into ice-water and extracted with ethyl acetate. The organic layer was dried and concentrated to dryness to give the title compound.
¹H NMR (CDCl₃) δ 7.18(s,1H), 6.95(s,2H), 4.16(s,3H), 2.69(s,3H), 2.31 (s,3H), 2.05(s,6H) ppm.

### PREPARATION I

### 7-Amino-1,5-dimethyl-3-(2,4,6-trimethyl-phenyl)-1H-pyrrolo[3,2-b]pyridine-6-carboxylic acid methyl ester

A mixture of 3-amino-1-methyl-4-(2,4,6-trimethylphenyl)-1H-pyrrole-2-carbonitrile (2 mmol), methyl acetoacetate (4 mmol) and SnCl₄ (4 mmol) in 1, 2-dichloroethane was heated at reflux for about six hours until all the starting material were consumed. The mixture was quenched with acetone, basified with saturated NaHCO₃, then filtered through Celite® . The filtrate was concentrated to dryness. The residue was quenched with water and extracted with chloroform. The chloroform layer was washed with brine, dried and concentrated to dryness to give 7-amino-1,5-dimethyl -3-(2,4,6-trimethylphenyl)-1H-pyrrolo[3,2-b]pyridine-6-carboxylic acid methyl ester. The product may be purified by trituration.
¹H NMR (CDCl₃) δ 6.91 (s,2H), 6.85(s,1H), 6.28(brs,2H), 4.10(s,3H), 3.89(s,3H), 2.62(s,3H), 2.28(s,3H), 2.09(s,6H) ppm.

### PREPARATION J

### 7-Bromo-1,5-dimethyl-3-(2,4,5-trimethyl-phenyl)-1H-pyrrolo[3,2-b]pyridine-6-carboxylic acid methyl ester

A mixture 7-amino-1, 5-dimethyl-3-(2, 4, 6-trimethylphenyl)-1H-pyrrolo[3, 2-b]pyridine-6-carboxylic acid methyl ester (1 mmol), n-butylnitrite (BuONO) (1.5 mmol) and CuBr₂ in acetonitrile was heated at 60-70°C until all starting material was consumed. The mixture was quenched with water and extracted with ethyl acetate. The organic layer was dried and concentrated to dryness to give the title compound.
¹H NMR (CDCl₃) δ 7.09(s,1H), 6.94(s,2H), 4.16(s,3H), 3.98(s,3H), 2.52(s,3H), 2.30(s,3H), 2.04(s,6H) ppm. 2,7-dibromo-1,5-dimethyl-3-(2,4,6-trimethyl-phenyl)-1H-pyrrolo[3,2-b]pyridine-6-carboxylic acid methyl ester was also produced as a minor component. ¹H NMR (CDCl₃) δ 7.00(s,2H), 4.45(s,3H), 4.03(s,3H), 2.51 (s,3H), 2.34(s,3H), 2.12(s,6H) ppm.

## Claims

1. A compound having a formula selected from the group consisting of: 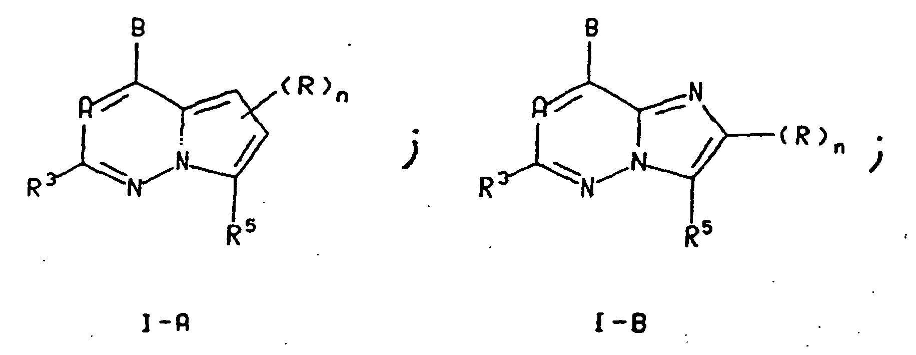 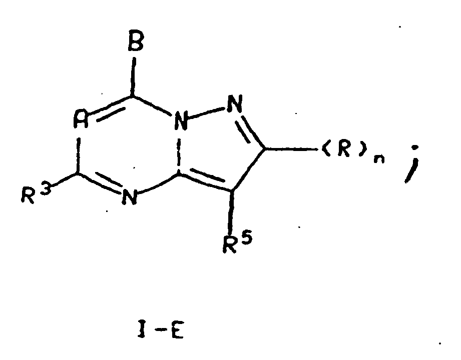 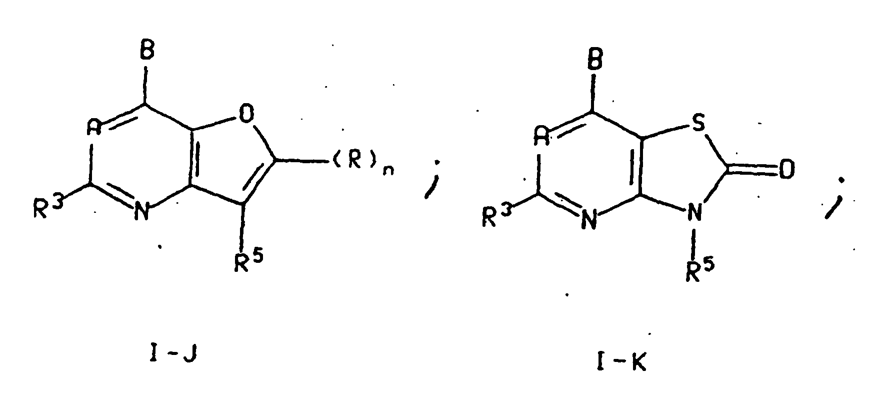 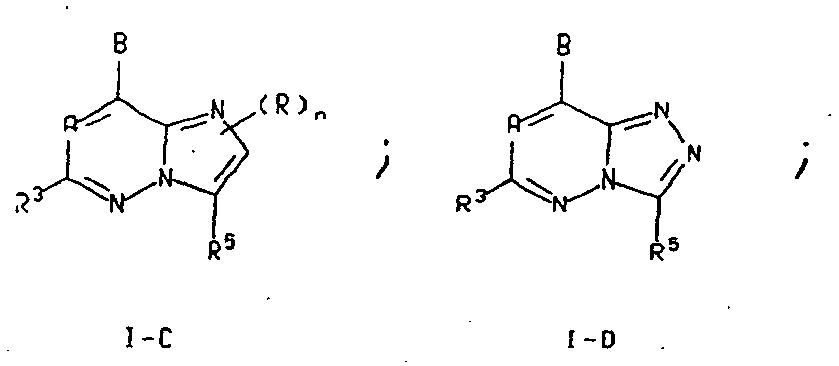 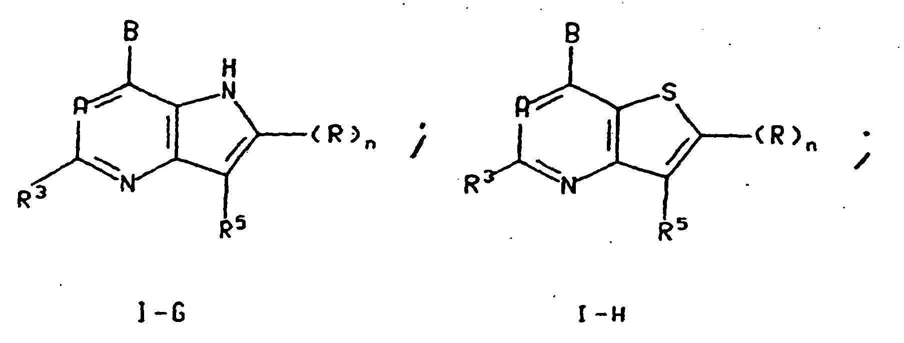 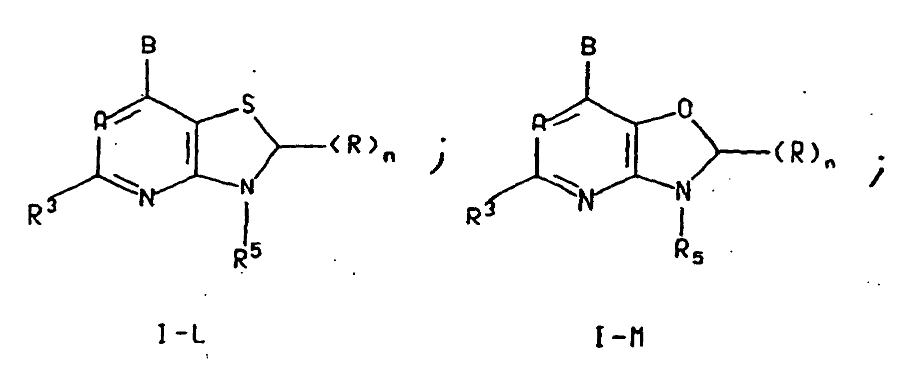 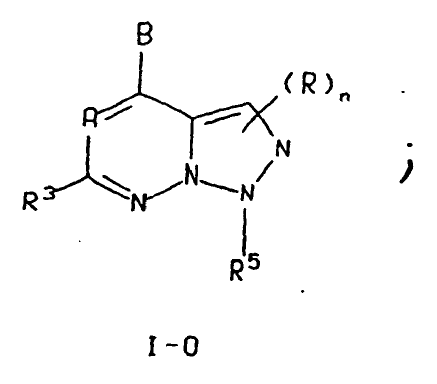 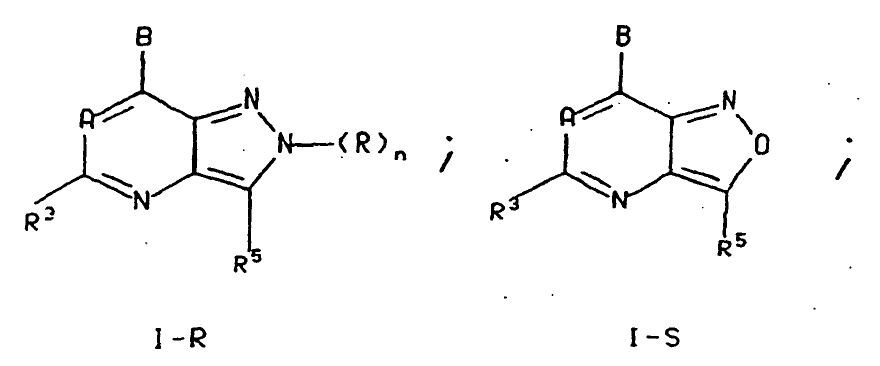 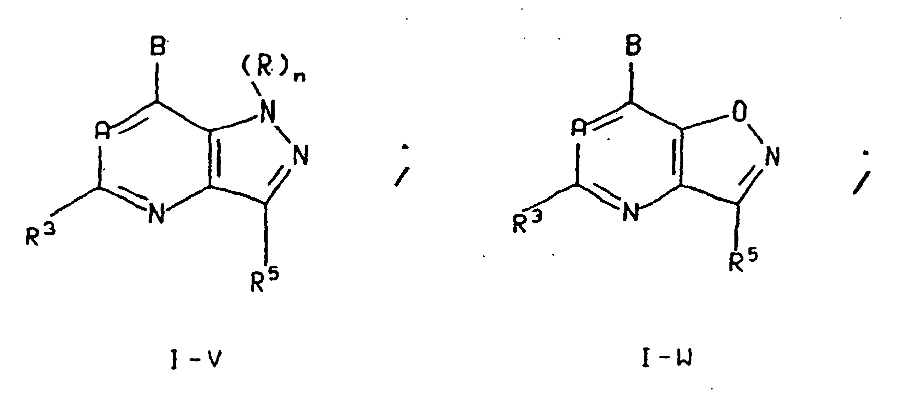 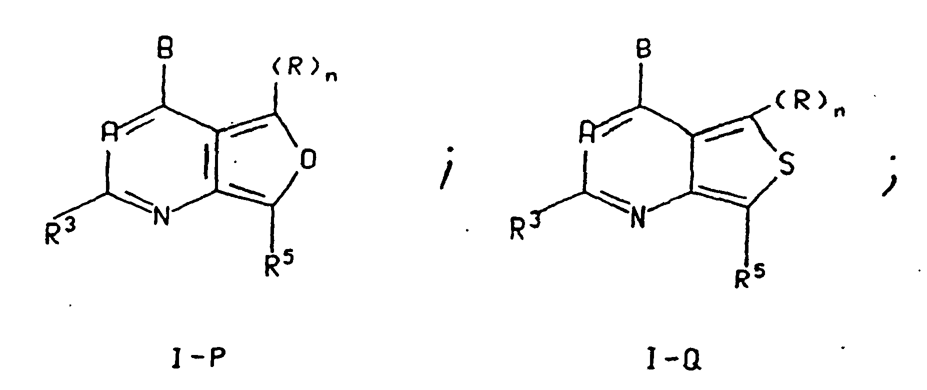 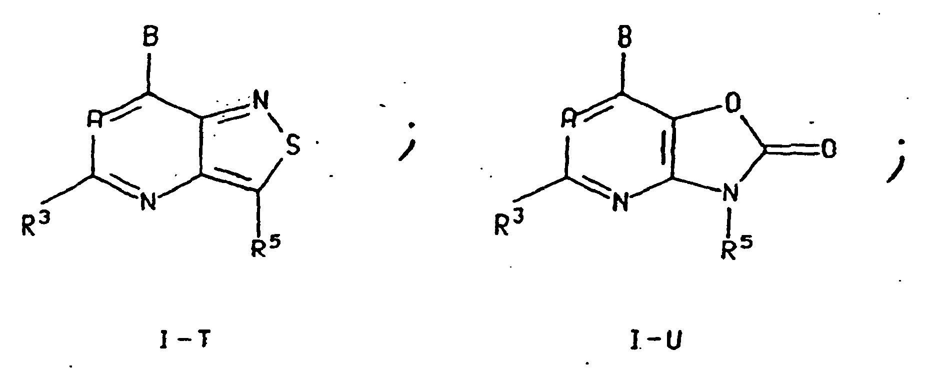 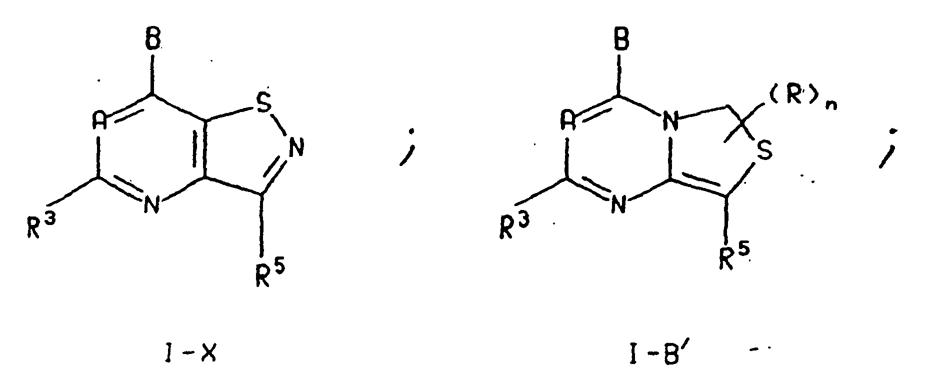 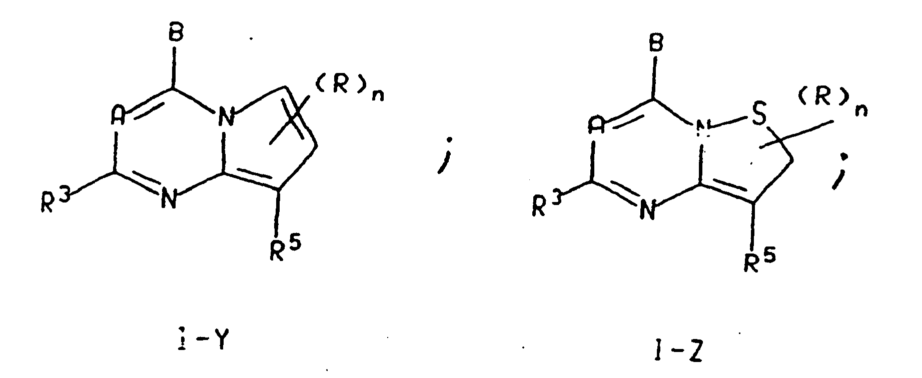 and 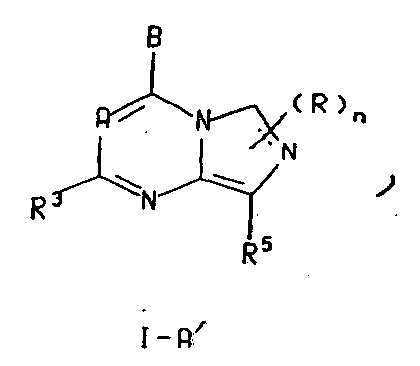 wherein
A is nitrogen or CR⁷;
B is -CHR¹R², -NR¹R², -NHCHR¹R², -OCHR¹R² or -SCHR¹R²;
R¹ is C₁-C₆ alkyl optionally substituted with one hydroxy, fluoro, CF₃, or (C₁-C₄ alkoxy), wherein the C₁-C₄ alkoxy group may optionally contain one double or triple bond;
R² is benzyl or C₁-C₆ alkyl which may optionally contain one double or triple bond, and wherein said C₁-C₆ alkyl and the phenyl moiety of said benzyl may optionally be substituted with one fluoro, CF₃, C₁-C₂ alkyl, C₁-C₂ alkoxy or chloro group;
R³ is hydrogen, C₁-C₄ alkyl, -O(C₁-C₄ alkyl), chloro, fluoro, bromo, iodo, (C₁-C₂ alkylene)-O-(C₁-C₂ alkyl), (C₁-C₂ alkylene)-OH or -S(C₁-C₄ alkyl);
R⁵ is phenyl, pyridyl, pyrazinyl, pyrimidyl, pyridazinyl and wherein each of the foregoing R⁵ groups is substituted with from one to four substituents R¹³ wherein one to three of said substituents may be selected, independently, from fluoro, chloro, C₁-C₆ alkyl and -O(C₁-C₆ alkyl) and one of said substituents may be selected from bromo, iodo, fonnyl, OH, (C₁-C₄ alkylene)-OH, -CN, -CF₃, NO₂, -NH₂, NH(C₁-C₄ alkyl), N(C₁-C₂ alkyl)(C₁₋C₆ alkyl), -OCO(C₁-C₄ alkyl), (C₁-C₄ alkylene)-O-(C₁-C₄ alkyl), -S(C₁-C₆ alkyl), (C₁-C₄ alkylene)-S-(C₁-C₄ alkyl), -C(=O)O(C₁-C₄ alkyl), -C(=O)(C₁-C₄ alkyl), -COOH,-SO₂NH(C₁-C₄ alkyl), -SO₂N(C₁-C₂ alkyl)(C₁-C₄ alkyl), -SO₂NH₂, -NHSO₂(C₁-C₄ alkyl),-S(C₁-C₆ alkyl) and -SO₂(C₁-C₆ alkyl), and wherein each of the C₁-C₄ alkyl and C₁-C₆ alkyl moieties in the foregoing R⁵ groups may optionally have one or two double bonds;
R⁷ is hydrogen, C₁-C₄ alkyl, halo (e.g., chloro, fluoro, iodo or bromo), hydroxy,-O(C₁-C₄ alkyl), -C(=O)(C₁-C₄ alkyl),-C(=O)O(C₁-C₄ alkyl), -OCF₃, -CF₃, -CH₂OH or-CH₂O(C₁-C₂ alkyl);
(R)ₙ represents from zero to two substituents, wherein when n is 1 and said (R)₁ group is attached to a carbon atom, (R)₁ is hydrogen, (C₁-C₆) alkyl, fluoro, chloro, bromo, iodo, hydroxy, cyano, amino, (C₁-C₂ alkylene)-OH, CF₃, CH₂SCH₃, nitro, O(C₁-C₄ alkyl),-N(C₁-C₄ alkyl)(C₁-C₂ alkyl), -S(C₁-C₄ alkyl), -CO(C₁-C₄ alkyl), -COH or -COO(C₁-C₄ alkyl); and wherein when n is 1 and said (R)₁ is attached to a nitrogen atom, (R)₁ is hydrogen or C₁-C₄ alkyl; and wherein when n is 2 and (R)₂ is attached to a carbon atom, (R)₂ is R^{a}R^{b}, wherein R^{a} is hydrogen, C₁-C₆ alkyl, fluoro, chloro, bromo, iodo, hydroxy, cyano, amino, (C₁-C₂ alkylene)-OH, CF₃, CH₂SCH₃, nitro, -O(C₁-C₄ alkyl), -N(C₁-C₄ alkyl)(C₁-C₂ alkyl), -S(C₁-C₄ alkyl), -CO(C₁-C₄ alkyl), -COH or -COO(C₁-C₄ alkyl); and R^{b} is hydrogen, methyl or ethyl;
and the pharmaceutically acceptable salts of such compounds.

2. A compound according to claim 1 wherein B is -NR¹R², -NHCHR¹R² or -OCHR¹R², and R¹ is C₁-C₆ alkyl, which may optionally be substituted with one fluoro or C₁-C₄ alkoxy group and which may optionally contain one double or triple bond; and R² is C₁-C₄ alkyl which may optionally contain one double or triple bond.

3. A compound according to claim 1 wherein R³ is methyl.

4. A compound according to claim 1 wherein R⁵ is di- or tri-substituted phenyl in which the two or three substitutents R¹³ are independently selected from fluoro, chloro C₁-C₄ alkyl, O-(C₁₋C₄ alkyl), and one of the two to three may be selected from (C₁-C₄ alkylene)-O-(C₁-C₄ alkyl), CF₃, CHO, (C₁-C₄ alkylene)-OH, cyano, bromo and iodo, wherein each of the forgoing (C₁-C₄) alkyl groups may optionally contain one double or triple bond.

5. A compound according to claim 1 wherein R³ is methyl, ethyl, chloro or methoxy.

6. A compound according to claim 1 wherein R⁵ is di- or tri-substituted pyridyl, or pyrimidyl in which the two or three substitutents are independently selected from fluoro, chloro C₁-C₄ alkyl, O-(C₁-C₄ alkyl), and one of the two to three may be selected from (C₁-C₄ alkylene)-O-(C₁-C₄ alkyl), CF₃, CHO, (C₁-C₄ alkylene)-OH, cyano, bromo and iodo, wherein each of the forgoing (C₁-C₄) alkyl groups may optionally contain one double or triple bond.

7. A compound according to claim 1 wherein A is N, CH or CCH₃.

8. A compound according to claim 1 wherein for the compounds of the formula I-E, (R)ₙ is hydrogen, CH₃ or C₂H₅.

9. A compound according to claim 1, having a formula selected from the group consisting of:
I-G where (R)₁ is CH₃ or C₂H₅; I-H where (R)₁ is H or CH₃; I-L where (R)₂ is (R^{a}R^{b}) and both R^{a} and R^{b} are H; I-M where (R)₂ is (R^{a}R^{b}) and both R^{a} and R^{b} are H; I-V where (R)₁ is CH₃; I-W; and I-X.

10. A compound according to claim 1 wherein B is -CHR¹R², -NHCHR¹R² or -OCHR¹R².

11. A pharmaceutical composition for the treatment, prevention or inhibition of (a) a disorder the treatment of which can be effected or facilitated by antagonizing CRF, disorders induced or facilitated by CRF, or (b) a disorder selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome, Crohn's disease; spastic colon; post operative ileus; ulcer; diarrhea; stress-induced fever; human immunodeficiency virus (H IV) infections; neurodegenerative diseases such as Alzheimer's disease, Parkinson's disease and Huntington's disease; gastrointestinal diseases; eating disorders such as anorexia and bulimia nervosa; hemorrhagic stress; chemical dependencies and addictions (eg., dependencies on alcohol, cocaine, heroin, benzodiazepines, or other drugs); drug and alcohol withdrawal symptoms; stress-induced psychotic episodes; euthyroid sick syndrome; syndrome of inappropriate antidiuretic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions (e.g., porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; hypertension; tachycardia; congestive heart failure; osteoporosis; premature birth; and hypoglycemia in a mammal, comprising an amount of a compound according to claim 1 that is effective in the treatment of such disorder, and a pharmaceutically acceptable carrier.

12. Use of a compound according to claim 1 in the manufacture of a medicament for the treatment, prevention or inhibition of a disorder selected from inflammatory disorders such as rheumatoid arthritis and osteoarthritis, pain, asthma, psoriasis and allergies; generalized anxiety disorder; panic; phobias; obsessive compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome, Crohn's disease; spastic colon; post operative ileus; ulcer; diarrhea; stress-induced fever; human immunodeficiency virus (HIV) infections; neurodegenerative diseases such as Alzheimer's disease, Parkinson's disease and Huntington's disease; gastrointestinal diseases; eating disorders such as anorexia and bulimia nervosa; hemorrhagic stress; chemical dependencies and addictions(eg., dependencies on alcohol, cocaine, heroin, benzodiazepines, or other drugs); drug and alcohol withdrawal symptoms; stressinduced psychotic episodes; euthyroid sick syndrome; syndrome of inappropriate antidiuretic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions(e.n., porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; hypertension; tachycardia; congestive heart failure; osteoporosis; premature birth; and hypoglycemia in a mammal.

13. A pharmaceutical composition for treating or preventing a disorder or condition, the treatment or prevention of which can be effected or facilitated by inhibiting CRH binding protein in a mammal, comprising a CRH binding protein inhibiting amount of a compound according to claim 1 and a pharmaceutically acceptable carrier.

14. A compound according to either of claims 8 and 9 wherein A is N or CH, R³ is methyl and (R)ₙ is, independently, hydrogen or methyl.

15. A compound according to claim 14, wherein R⁵ is di- or tri-substituted phenyl, wherein the two or three substitutents are independently selected from fluoro, chloro C₁-C₄ alkyl, O-(C₁₋C₄ alkyl), and one of the two to three may be selected from (C₁-C₄ alkylene)-O-(C₁-C₄ alkyl), CF₃, CHO, (C₁-C₄ alkylene)-OH, cyano, bromo and iodo, wherein each of the forgoing (C₁-C₄) alkyl groups may optionally contain one double or triple bond

16. A compound according to claim 1 wherein said compound is:
7-(1-ethyl-propoxy)-5-methyl-3-(2,4,6-trimethyl-phenyl)-pyrazolo[1,5-a]pyrimidine;
[2,5-Dimethyl-3-(2,4,6-trimethyl-phenyl)-pyrazolo[1,5-a]pyrimidin-7-yl]-(1-ethylpropyl)-amine;
(1-Ethyl-propyl)-[5-methyl-3-(2,4,6-trimethyl-phenyl)-pyrazolo[1,5-a]pyrimidin-7-yl]- amine;
7-(1-Ethyl-propoxy)-2,5-dimethyl-3-(2,4,6-trimethyl-phenyl)-pyrazolo[1,5-a]pyrimidine;
[2,5-Dimethyl-3-(2,4,6-trimethyl-phenyl)-pyrazolo[1,5-a]pyrimidin-7-yl]-ethyl-propylamine;
or a pharmaceutically acceptable salt of such a compound.

## Patentansprüche

1. Verbindung mit einer Formel, die aus der Gruppe ausgewählt ist, bestehend aus: 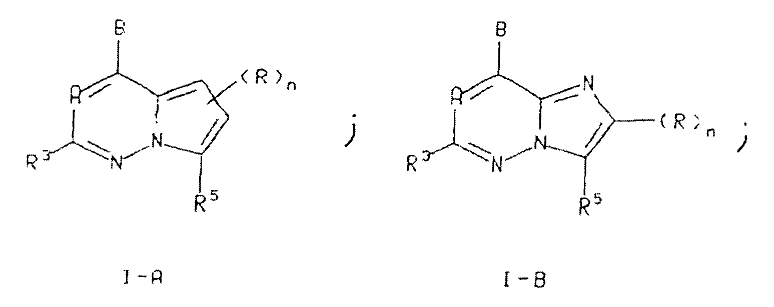 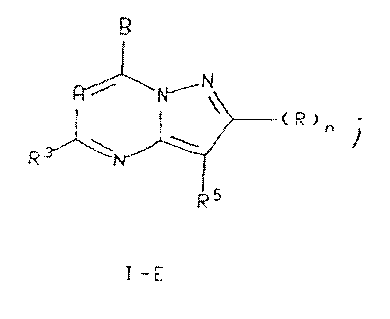 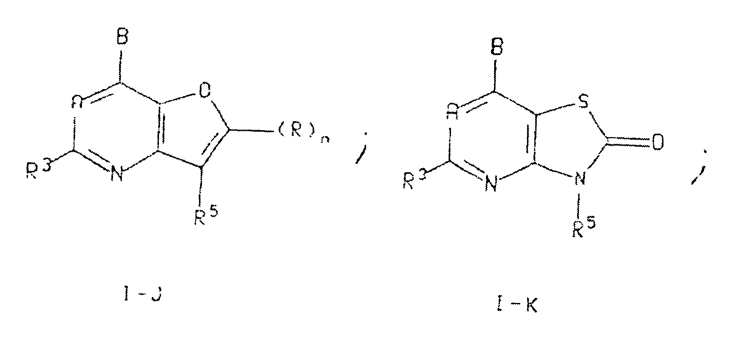 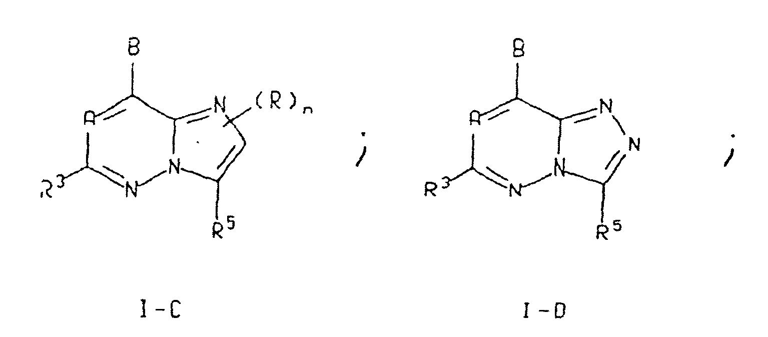 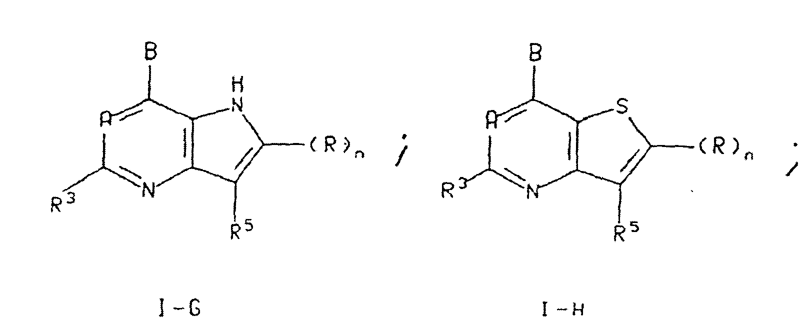 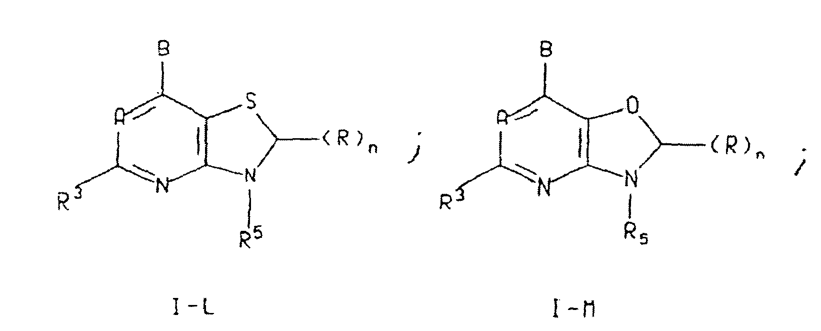 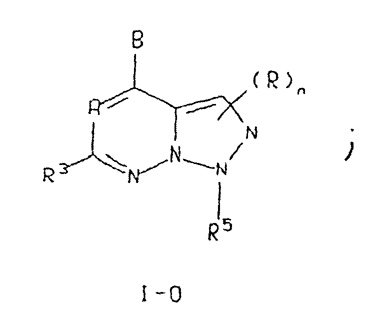 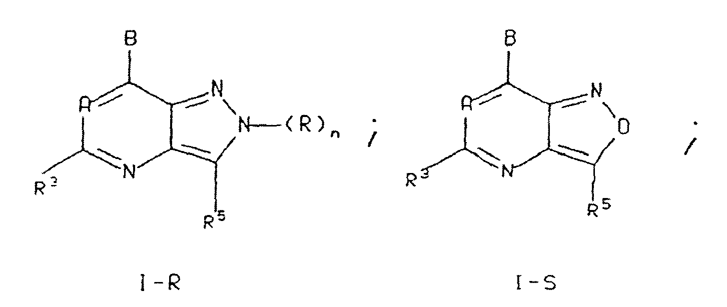 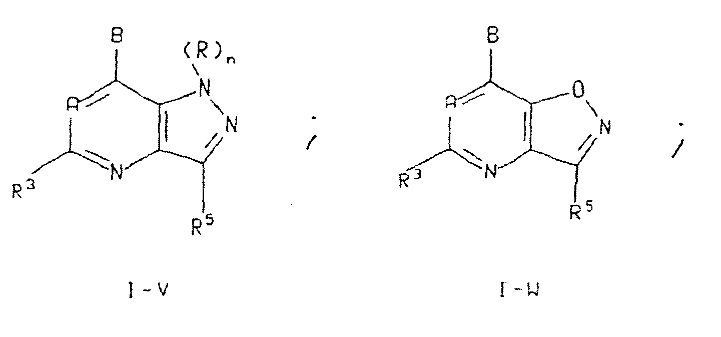 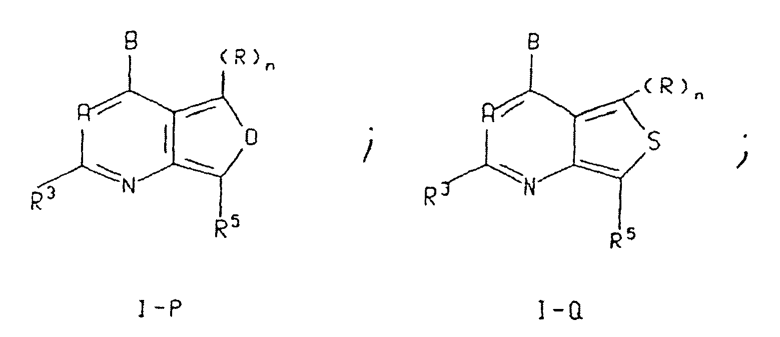 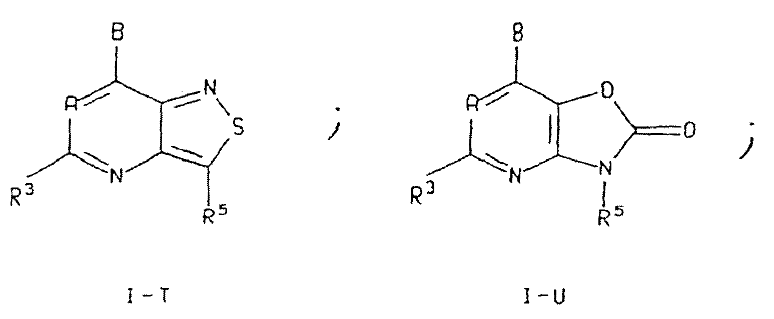 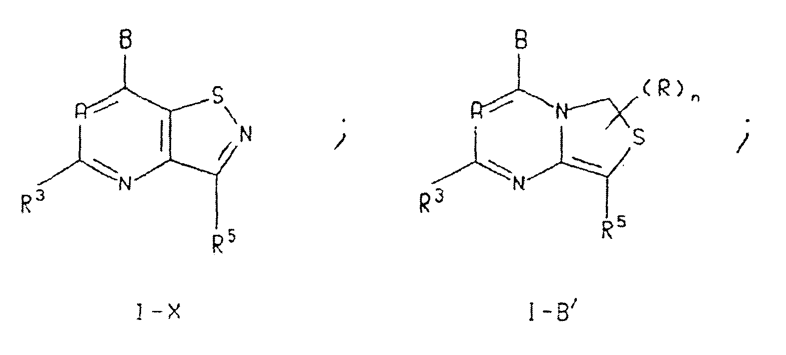 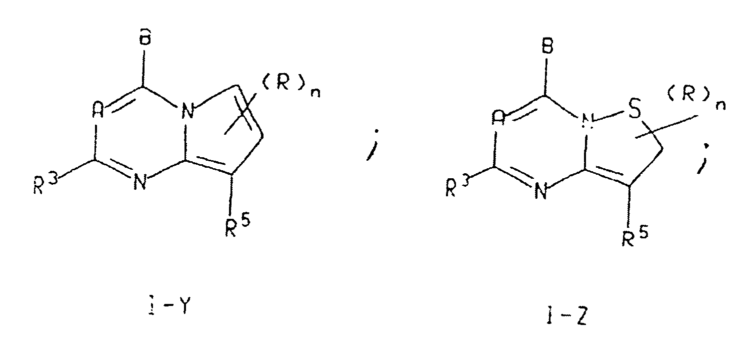 und 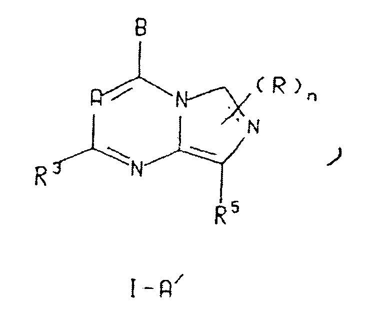 worin
A für Stickstoff oder CR⁷ steht;
B für -CHR¹R², -NR¹R², -NHCHR¹R², -OCHR¹R² oder -SCHR¹R² steht;
R¹ C₁-C₆-Alkyl, optional substituiert mit einem Hydroxy, Fluoro, CF₃ oder (C₁-C₄-Alkoxy) darstellt, worin die C₁-C₄-Alkoxygruppe optional eine Doppel- oder Dreifachbindung enthalten kann;
R² Benzyl oder C₁-C₆-Alkyl darstellt, das optional eine Doppel- oder Dreifachbindung enthalten kann und worin das genannte C₁-C₆-Alkyl und die Phenylkomponente vom genannten Benzyl optional mit einer Fluoro-, CF₃-, C₁-C₂-Alkyl-, C₁-C₂-Alkoxy- oder Chloro-Gruppe substituiert sein können;
R³ Wasserstoff, C₁-C₄-Alkyl, -O-(C₁-C₄-Alkyl), Chloro, Fluoro, Bromo, Iodo, (C₁-C₂-Alkylen)-O-(C₁-C₂₋alkyl), (C₁-C₂-Alkylen)-OH oder -S-(C₁-C₄-Alkyl) darstellt;
R⁵ Phenyl, Pyridyl, Pyrazinyl, Pyrimidyl, Pyridazinyl darstellt und worin jede der vorstehenden R⁵-Gruppen mit von einem bis vier Substituenten R¹³ substituiert ist, worin einer bis drei der genannten Substituenten unabhängig aus Fluoro, Chloro, C₁-C₆-Alkyl und -O-(C₁-C₆-Alkyl) ausgewählt sein kann/können und einer der genannten Substituenten aus Bromo, Iodo, Formyl, OH, (C₁₋C₄-Alkylen) -OH, -CN, -CF₃, -NO₂, -NH₂, -NH-(C₁-C₄₋Alkyl), -N- (C₁-C₂-Alkyl)-(C₁-C₆-Alkyl), -OCO-(C₁-C₄₋Alkyl), (C₁-C₄-Alkylen)-O-(C₁-C₄-Alkyl), -S-(C₁-C₆-Alkyl), (C₁-C₄-Alkylen)-S-(C₁-C₄-Alkyl),-C(=O)-O-(C₁-C₄₋Alkyl), -C(=O)-(C₁-C₄-Alkyl), -COOH, -SO₂NH-(C₁-C₄₋Alkyl), -SO₂N-(C₁-C₂-Alkyl)-(C₁-C₄₋alkyl), -SO₂NH₂, -NHSO₂-(C₁-C₄-Alkyl), -S-(C₁-C₆-Alkyl) und -SO₂-(C₁-C₆-Alkyl) ausgewählt sein kann, und worin jede der C₁-C₄-Alkyl- und C₁-C₆-Alkylkomponenten in den vorstehenden R⁵-Gruppen optional eine oder zwei Doppelbindung(en)aufweisen kann;
R⁷ Wasserstoff, C₁-C₄-Alkyl, Halo, Hydroxy, -O- (C₁₋C₄-Alkyl), -C(=O)-(C₁-C₄-Alkyl),-C(=O)-O-(C₁-C₄₋Alkyl), -OCF₃, -CF₃, -CH₂OH- oder -CH₂O-(C₁-C₂-Alkyl) darstellt;
(R)ₙ von null bis zwei Substituenten darstellt, worin, wenn n für 1 steht und die genannte (R)₁-Gruppe an ein Kohlenstoffatom gebunden ist, (R)₁ Wasserstoff, (C₁-C₆)-Alkyl, Fluoro, Chloro, Bromo, Iodo, Hydroxy, Cyano, Amino, (C₁-C₂-Alkylen)-OH, CF₃, CH₂SCH₃, Nitro, O-(C₁-C₄-Alkyl), -N-(C₁-C₄-Alkyl)-(C₁-C₂-Alkyl), -S-(C₁₋C₄-Alkyl), -CO-(C₁-C₄-Alkyl), -COH- oder -COO-(C₁-C₄₋Alkyl) darstellt; und worin, wenn n für 1 steht und genanntes (R)₁ an ein Stickstoffatom gebunden ist, (R)₁ Wasserstoff oder C₁-C₄-Alkyl darstellt; und worin, wenn n für 2 steht und (R)₂ an ein Kohlenstoffatom gebunden ist, (R)₂ für R^{a}R^{b} steht, worin R^{a} Wasserstoff, C₁-C₆₋Alkyl, Fluoro, Chloro, Bromo, Iodo, Hydroxy, Cyano, Amino, (C₁-C₂-Alkylen)-OH, CF₃, CH₂SCH₃, Nitro, -O-(C₁₋C₄-Alkyl), -N-(C₁-C₄-Alkyl)-(C₁-C₂-Alkyl), -S-(C₁-C₄₋Alkyl), -CO-(C₁-C₄-Alkyl), -COH- oder -COO-(C₁-C₄-Alkyl) darstellt; und R^{b} Wasserstoff, Methyl oder Ethyl darstellt;
und die pharmazeutisch verträglichen Salze solcher Verbindungen.

2. Verbindung nach Anspruch 1, worin B für -NR¹R², -NHCHR¹R² oder -OCHR¹R² steht, und R¹ für C₁₋C₆-Alkyl steht, das optional mit einer Fluoro- oder C₁-C₄-Alkoxygruppe substituiert sein kann und das optional eine Doppel- oder Dreifachbindung enthalten kann; und R² für C₁-C₄-Alkyl steht, das optional eine Doppel- oder Dreifachbindung enthalten kann.

3. Verbindung nach Anspruch 1 worin R³ Methyl darstellt.

4. Verbindung nach Anspruch 1, worin R⁵ di- oder trisubstituiertes Phenyl darstellt, worin die zwei oder drei Substituenten R¹³ unabhängig aus Fluoro, Chloro, C₁-C₄-Alkyl, O-(C₁-C₄-Alkyl) ausgewählt sind und einer der zwei bis drei aus (C₁-C₄-Alkylen)-O-(C₁-C₄-Alkyl), CF₃, OCF₃, CHO, (C₁-C₄-Alkylen) -OH, Cyano, Bromo und Iodo ausgewählt sein kann, worin jede der vorstehenden (C₁-C₄)-Alkylgruppen optional eine Doppel- oder Dreifachbindung enthalten kann.

5. Verbindung nach Anspruch 1, worin R³ Methyl, Ethyl, Chloro oder Methoxy darstellt.

6. Verbindung nach Anspruch 1, worin R⁵ di- oder trisubstituiertes Pyridyl oder Pyrimidyl darstellt, worin die zwei oder drei Substituenten unabhängig aus Fluoro, Chloro, C₁-C₄-Alkyl, O-(C₁-C₄-Alkyl) ausgewählt sind und einer der zwei bis drei aus (C₁-C₄-Alkylen)-O-(C₁-C₄-Alkyl), CF₃, OCF₃, CHO, (C₁-C₄-Alkylen)-OH, Cyano, Bromo und Iodo ausgewählt sein kann, worin jede der vorstehenden (C₁-C₄)-Alkylgruppen optional eine Doppel- oder Dreifachbindung enthalten kann.

7. Verbindung nach Anspruch 1, worin A für N, CH oder CCH₃ steht.

8. Verbindung nach Anspruch 1, worin für die Verbindungen der Formel I-E (R)ₙ für Wasserstoff, CH₃ oder C₂H₅ steht.

9. Verbindung nach Anspruch 1 mit einer Formel, die aus der Gruppe ausgewählt ist, bestehend aus:
I-G, worin (R)₁ für CH₃ oder C₂H₅ steht; I-H, worin (R)₁ für H oder CH₃ steht; I-L, worin (R) für (R^{a}R^{b}) steht und sowohl R^{a} als auch R^{b} für H stehen; I-M, worin (R)₂ für (R^{a}R^{b}) steht und sowohl R^{a} als auch R^{b} für H stehen; I-V, worin (R)₁ für CH₃ steht; I-W; und I-X.

10. Verbindung nach Anspruch 1, worin B für-CHR¹R², -NHCHR¹R² oder -OCHR¹R² steht.

11. Pharmazeutische Zusammensetzung zur Behandlung, Prävention oder Inhibition von (a) einer Erkrankung, deren Behandlung durch den antagonistisch wirkenden CRF bewirkt oder gefördert werden kann, Erkrankungen, die durch CRF induziert oder gefördert werden, oder (b) eine Erkrankung, die aus Folgendem ausgewählt ist: Entzündungserkrankungen, wie zum Beispiel rheumatoider Arthritis und Osteoarthritis, Schmerzen, Asthma, Psoriasis und Allergien; generalisierter Angststörung; Panikstörung; Phobien; Zwangsstörung; posttraumatischer Belastungsstörung; belastungsinduzierten Schlafstörungen; Schmerzwahrnehmung, wie zum Beispiel Fibromyalgie; affektiven Störungen, wie zum Beispiel Depression, einschließlich majorer Depression, majorer Depression, einzelne Episode, rezidivierender depressiver Störung, durch körperliche Misshandlung eines Kindes induzierter Depression, mit prämenstruellem Syndrom assoziierten affektiven Störungen und postpartaler Depression; Dysthymia; bipolaren Störungen; Zyklothymia; Chronischem Erschöpfungssyndrom; belastungsinduziertem Kopfschmerz; Krebs; Reizdarm-Syndrom, Morbus Crohn; spastischem Kolon; postoperativem Ileus; Ulkus; Diarrhöe; belastungsinduzierter erhöhter Körpertemperatur; Human Immunodeficiency Virus-Infektionen (HIV-Infektionen); neurodegenerativen Erkrankungen, wie zum Beispiel Alzheimer-Krankheit, Parkinson-Krankheit und Huntington-Krankheit; gastrointestinalen Erkrankungen; Essstörungen, wie zum Beispiel Anorexia und Bulimia nervosa; hämorrhagischem Stress; Substanzabhängigkeit und -sucht (z. B. Abhängigkeit von Alkohol, Kokain, Heroin, Benzodiazepinen oder anderen Arzneimitteln/Drogen); Arzneimittel/Drogen- und Alkoholentzugssymptomen; belastungsinduzierten Episoden mit psychotischen Symptomen; Euthyroid Sick Syndrome; inadäquatem ADH-Sekretionssyndrom; Adipositas; Infertilität; Kopftraumata; Rückenmarktrauma; Ischämie-induzierter neuronaler Schädigung (z. B. zerebraler Ischämie, wie zum Beispiel zerebral-hippokampaler Ischämie); excitotoxischer neuronaler Schädigung; Epilepsie; Schlaganfall; Immundysfunktionen, einschließlich belastungsinduzierten Immundysfunktionen (z. B. porzinem Stress-Syndrom, bovinem Shipping Fever, equiner paroxysmaler Fibrillation und bei in Massen auf beengtem Raum lebenden Hühnern induzierter Dysfunktionen, Scherstress bei Schafen oder durch Mensch-Tier-Interaktion bedingtem Stress bei Hunden); Muskelspasmen; Harninkontinenz; seniler Demenz vom Alzheimer Typ; Multiinfarktdemenz; amyotropher Lateralsklerose; Hypertonie; Tachykardie; dekompensierter Herzinsuffizienz; Osteoporose; Frühgeburt; und Hypoglykämie in einem Säuger, umfassend eine Menge einer Verbindung nach Anspruch 1, die in der Behandlung einer solchen Erkrankung wirksam ist und einen pharmazeutisch verträglichen Träger.

12. Verwendung einer Verbindung nach Anspruch 1 bei der Herstellung eines Arzneimittels zur Behandlung, Prävention oder Inhibition einer Erkrankung, die aus Folgendem ausgewählt ist: Entzündungserkrankungen, wie zum Beispiel rheumatoider Arthritis und Osteoarthritis, Schmerzen, Asthma, Psoriasis und Allergien; generalisierter Angststörung; Panikstörung; Phobien; Zwangsstörung; posttraumatischer Belastungsstörung; belastungsinduzierten Schlafstörungen; Schmerzwahrnehmung, wie zum Beispiel Fibromyalgie; affektiven Störungen, wie zum Beispiel Depression, einschließlich majorer Depression, majorer Depression, einzelne Episode, rezidivierender depressiver Störung, durch körperliche Misshandlung eines Kindes induzierter Depression, mit prämenstruellem Syndrom assoziierten affektiven Störungen und postpartaler Depression; Dysthymia; bipolaren Störungen; Zyklothymia; Chronischem Erschöpfungssyndrom; belastungsinduziertem Kopfschmerz; Krebs; Reizdarm-Syndrom, Morbus Crohn; spastischem Kolon; postoperativem Ileus; Ulkus; Diarrhöe; belastungsinduzierter erhöhter Körpertemperatur; Human Immunodeficiency Virus-Infektionen (HIV-Infektionen); neurodegenerativen Erkrankungen, wie zum Beispiel Alzheimer-Krankheit, Parkinson-Krankheit und Huntington-Krankheit; gastrointestinalen Erkrankungen; Essstörungen, wie zum Beispiel Anorexia und Bulimia nervosa; hämorrhagischem Stress; Substanzabhängigkeit und -sucht (z. B. Abhängigkeit von Alkohol, Kokain, Heroin, Benzodiazepinen oder anderen Arzneimitteln/Drogen); Arzneimittel/Drogen- und Alkoholentzugssymptomen; belastungsinduzierten Episoden mit psychotischen Symptomen; Euthyroid Sick Syndrome; inadäquatem ADH-Sekretionssyndrom; Adipositas; Infertilität; Kopftraumata; Rückenmarktrauma; Ischämie-induzierter neuronaler Schädigung (z. B. zerebraler Ischämie, wie zum Beispiel zerebral-hippokampaler Ischämie); excitotoxischer neuronaler Schädigung; Epilepsie; Schlaganfall; Immundysfunktionen, einschließlich belastungsinduzierten Immundysfunktionen (z. B. porzinem Stress-Syndrom, bovinem Shipping Fever, equiner paroxysmaler Fibrillation und bei in Massen auf beengtem Raum lebenden Hühnern induzierter Dysfunktionen, Scherstress bei Schafen oder durch Mensch-Tier-Interaktion bedingtem Stress bei Hunden); Muskelspasmen; Harninkontinenz; seniler Demenz vom Alzheimer Typ; Multiinfarktdemenz; amyotropher Lateralsklerose; Hypertonie; Tachykardie; dekompensierter Herzinsuffizienz; Osteoporose; Frühgeburt; und Hypoglykämie in einem Säuger.

13. Pharmazeutische Zusammensetzung zur Behandlung oder Prävention einer Erkrankung oder eines Zustands, deren/dessen Behandlung oder Prävention durch Inhibition des CRH-bindenden Proteins in einem Säuger bewirkt oder gefördert werden kann, umfassend eine das CRH-bindende Protein inhibierende Menge einer Verbindung nach Anspruch 1 und einen pharmazeutisch verträglichen Träger.

14. Verbindung nach einem der Ansprüche 8 und 9, worin A für N oder CH steht, R³ Methyl darstellt und (R)ₙ unabhängig Wasserstoff oder Methyl darstellt.

15. Verbindung nach Anspruch 14, worin R⁵ di- oder trisubstituiertes Phenyl darstellt, worin die zwei oder drei Substituenten unabhängig aus Fluoro, Chloro, C₁₋C₄-Alkyl, -O-(C₁-C₄-Alkyl) ausgewählt sind, und einer der zwei bis drei aus (C₁-C₄-Alkylen)-O-(C₁-C₄-Akyl), CF₃, OCF₃, CHO, (C₁-C₄-Alkylen)-OH, Cyano, Bromo und Iodo ausgewählt werden kann, worin jede der vorstehenden (C₁-C₄)-Alkylgruppen optional eine Doppel- oder Dreifachbindung enthalten kann.

16. Verbindung nach Anspruch 1, worin die genannte Verbindung folgende darstellt:
7-(1-Ethyl-propoxy)-5-methyl-3-(2,4,6-trimethylphenyl)-pyrazolo[1,5-a]pyrimidin;
[2,5-Dimethyl-3-(2,4,6-trimethyl-phenyl)-pyrazolo[1,5-a]pyrimidin-7-yl]-(1-ethylpropyl)-amin;
(1-Ethyl-propyl)-[5-methyl-3-(2,4,6-trimethylphenyl)-pyrazolo[1,5-a]pyrimidin-7-yl]-amin;
7-(1-Ethyl-propoxy)-2,5-dimethyl-3-(2,4,6-trimethyl-phenyl)-pyrazolo[1,5-a]pyrimidin;
[2,5-Dimethyl-3-(2,4,6-trimethyl-phenyl)-pyrazolo[1,5-a]pyrimidin-7-yl]-ethyl-propylamin;
oder ein pharmazeutisch verträgliches Salz von solch einer Verbindung.

## Revendications

1. Composé répondant à une formule sélectionnée parmi le groupe consistant en : 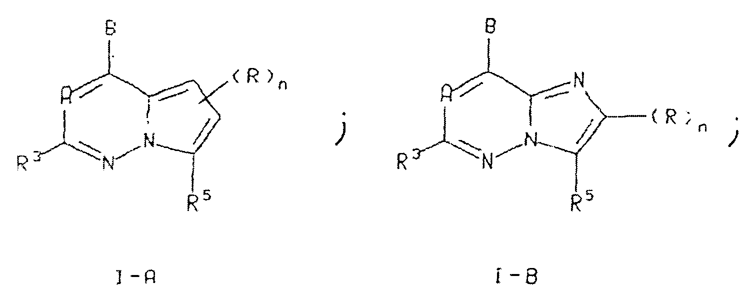 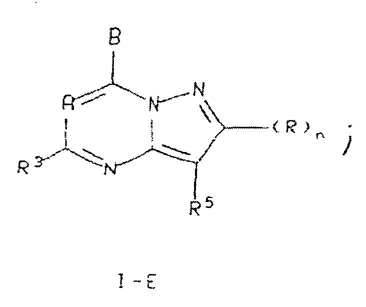 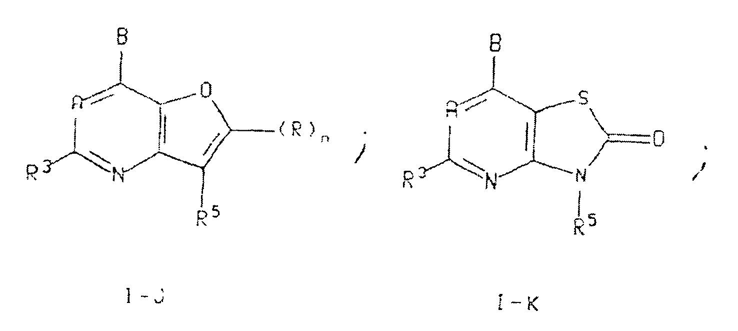 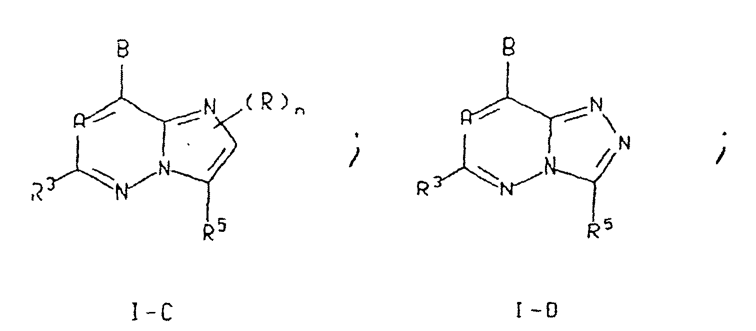 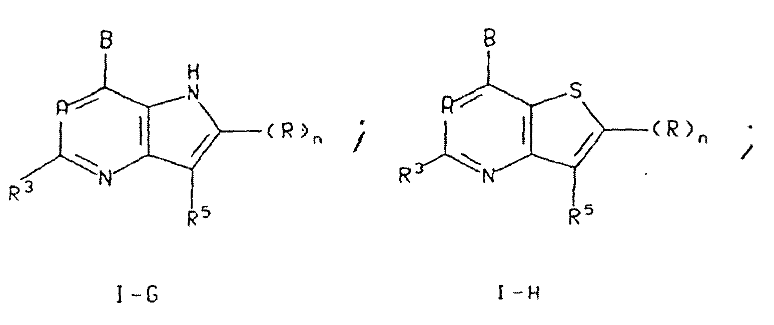 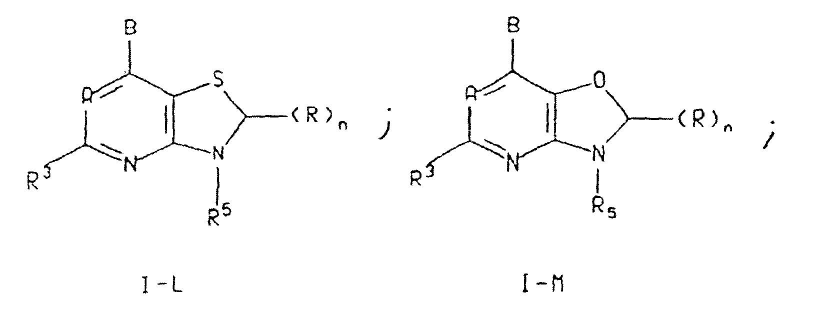 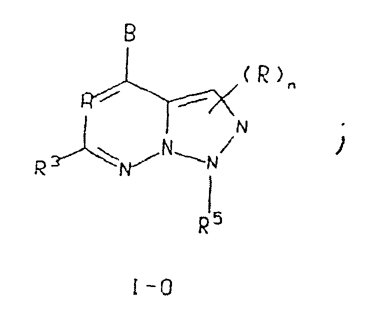 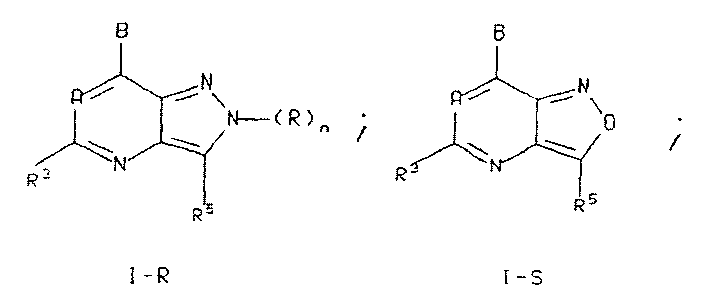 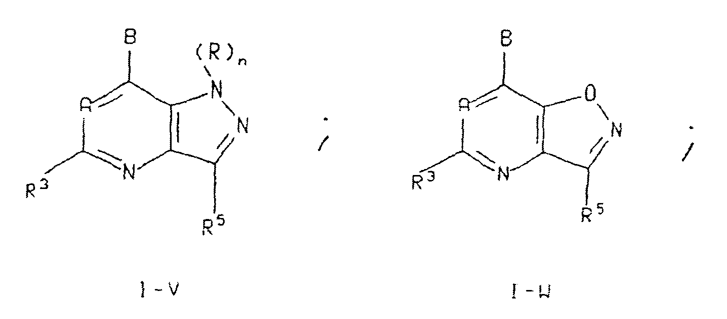 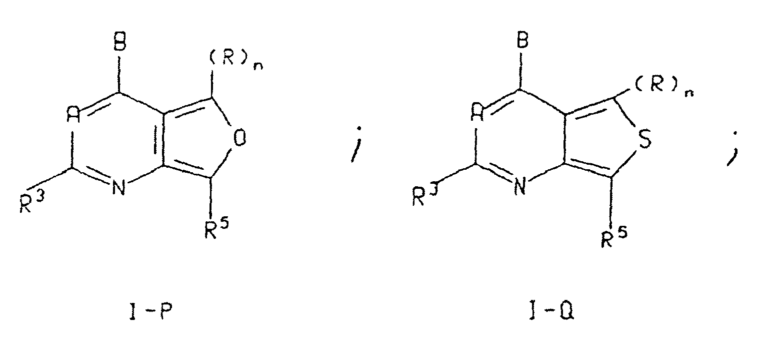 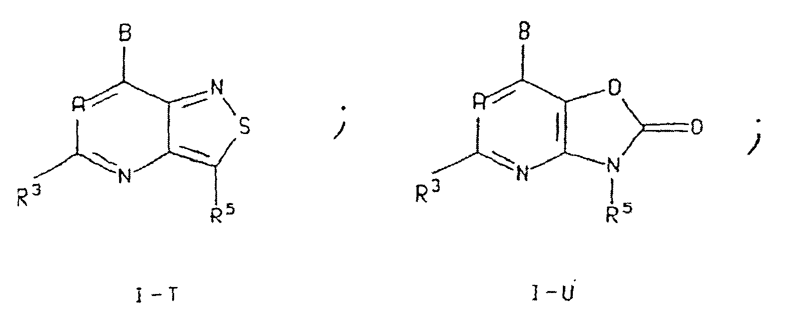 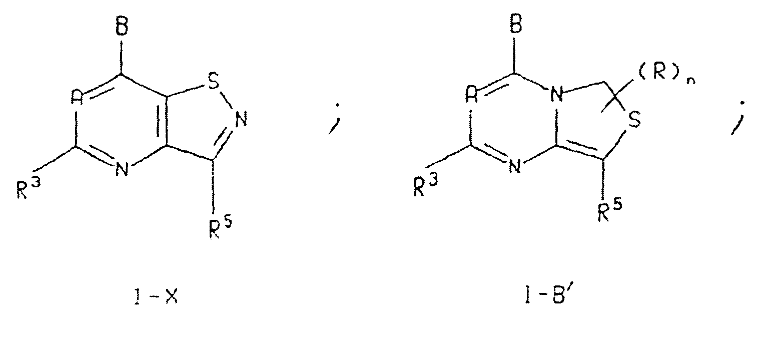 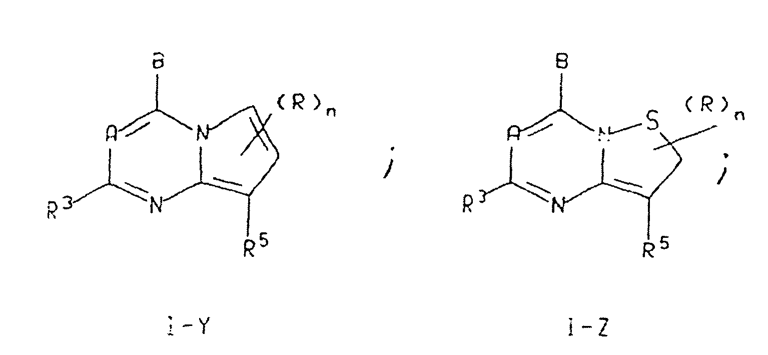 et 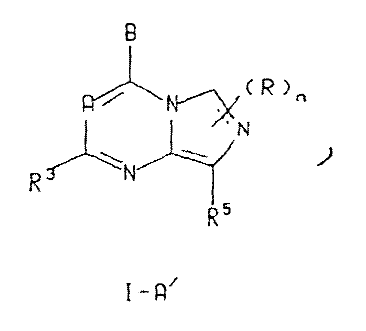 où
A représente un atome d'azote ou CR⁷ ;
B représente -CHR¹R², -NR¹R², -NHCHR¹R², -OCHR¹R² ou -SCHR¹R² ;
R¹ représente un groupement alkyle en C₁-C₆ qui est éventuellement substitué par un groupement hydroxy, fluoro, CF₃ ou alkoxy en C₁-C₄, ledit groupement alkoxy en C₁-C₄ contenant en option une liaison double ou triple ;
R² représente un groupement benzyle ou un groupement alkyle en C₁-C₆ contenant en option une liaison double ou triple, ledit groupement alkyle en C₁-C₆ et le fragment phényle dudit groupement benzyle étant éventuellement substitués par un groupement fluoro, CF₃, alkyle en C₁-C₂, alkoxy en C₁-C₂ ou chloro ;
R³ représente un atome d'hydrogène ou un groupement alkyle en C₁-C₄, -O(alkyle en C₁-C₄), chloro, fluoro, bromo, iodo, (alkylène en C₁-C₂)-O-(alkyle en C₁-C₂), (alkylène en C₁-C₂)-OH ou -S (alkyle en C₁-C₄) ;
R⁵ représente un groupement phényle, pyridyle, pyrazinyle, pyrimidyle, pyridazinyle, chacun des groupements R⁵ qui précèdent portant de un à quatre substituants R¹³ tels que de un à trois desdits substituants peuvent être indépendamment sélectionnés parmi un groupement fluoro, chloro, alkyle en C₁-C₆ et -O(alkyle en C₁-C₆) et un desdits substituants peut être sélectionné parmi un groupement bromo, iodo, formyle, OH, (alkylène en C₁-C₄)-OH, -CN, -CF₃, -NO₂,-NH₂, -NH(alkyle en C₁-C₄), -N (alkyle en C₁-C₂) (alkyle en C₁-C₆), -OCO(alkyle en C₁-C₄), (alkylène en C₁-C₄)-O-(alkyle en C₁-C₄), -S (alkyle en C₁-C₆), (alkylène en C₁₋C₄)-S-(alkyle en C₁-C₄), -C(=O)O(alkyle en C₁-C₄),-C(=O)(alkyle en C₁-C₄), -COOH, -SO₂NH(alkyle en C₁-C₄), -SO₂N (alkyle en C₁-C₂) (alkyle en C₁-C₄), -SO₂NH₂,-NHSO₂(alkyle en C₁-C₄), -S(alkyle en C₁-C₆) et-SO₂(alkyle en C₁-C₆), chacun des fragments alkyle en C₁₋C₄ et alkyle en C₁-C₆ des groupements R⁵ qui précèdent contenant en option une ou deux liaisons doubles ;
R⁷ représente un atome d'hydrogène ou un groupement alkyle en C₁-C₄, halo, hydroxy, -O(alkyle en C₁-C₄), -C(=O)(alkyle en C₁-C₄), -C(=O)O(alkyl en C₁-C₄), -OCF₃, -CF₃, -CH₂OH ou -CH₂O(alkyle en C₁-C₂);
(R)ₙ représente de zéro à deux substituants, tels que quand n est égal à 1 et ledit groupement (R)₁ est porté par un atome de carbone, (R)₁ représente un atome d'hydrogène ou un groupement alkyle en C₁-C₆, fluoro, chloro, bromo, iodo, hydroxy, cyano, amino, (alkylène en C₁-C₂)-OH, CF₃, CH₂SCH₃, nitro, O(alkyle en C₁-C₄),-N(alkyle en C₁-C₄)(alkyle en C₁-C₂), -S(alkyle en C₁-C₄) -CO(alkyle en C₁-C₄), -COH ou -COO(alkyle en C₁-C₄) ;ou tels que quand n est égal à 1 et ledit groupement (R)₁ est porté par un atome d'azote, (R)₁ représente un atome d'hydrogène ou un groupement alkyle en C₁-C₄ ; et tels que quand n est égal à 2 et ledit groupement (R)₂ est porté par un atome de carbone, (R)₂ représente R^{a}R^{b}, où R^{a} représente un atome d'hydrogène ou un groupement alkyle en C₁-C₆, fluoro, chloro, bromo, iodo, hydroxy, cyano, amino, (alkylène en C₁-C₂) -OH, CF₃, CH₂SCH₃, nitro, -O(alkyle en C₁-C₄), -N (alkyle en C₁-C₄) (alkyle en C₁-C₂), -S(alkyle en C₁-C₄), -CO(alkyle en C₁-C₄),-COH ou -COO(alkyle en C₁-C₄) ; et R^{b} représente un atome d'hydrogène ou un groupement méthyle ou éthyle ;
et les sels pharmaceutiquement acceptables de ces composés.

2. Composé selon la revendication 1, où B représente -NR¹R², -NHCHR¹R² ou -OCHR¹R² et R¹ représente un groupement alkyle en C₁-C₆ qui est éventuellement substitué par un groupement fluoro ou alkoxy en C₁-C₄ et qui peut en option contenir un liaison double ou triple ; et R² représente un groupement alkyle en C₁-C₄ qui contient en option une liaison double ou triple.

3. Composé selon la revendication 1, où R³ représente un groupement méthyle.

4. Composé selon la revendication 1, où R⁵ représente un groupement phényle di- ou trisubstitué dans lequel les deux ou les trois substituants R¹³ sont indépendamment sélectionnés parmi un groupement fluoro, chloro, alkyle en C₁-C₄ et O-(alkyle en C₁-C₄) et un des deux ou trois desdits substituants peut être sélectionné parmi un groupement (alkylène en C₁-C₄)-O-(alkyle en C₁-C₄), CF₃, OCF₃, CHO, (alkylène en C₁-C₄) -OH, cyano, bromo et iodo, chacun des groupements alkyles en C₁-C₄ qui précèdent contenant en option une liaison double ou triple.

5. Composé selon la revendication 1, où R³ représente un groupement méthyle, éthyle, chloro ou méthoxy.

6. Composé selon la revendication 1, où R⁵ représente un groupement pyridyle ou pyrimidyle di- ou trisubstitué dans lequel les deux ou les trois substituants sont indépendamment sélectionnés parmi un groupement fluoro, chloro, alkyle en C₁-C₄ et O-(alkyle en C₁-C₄) et un des deux ou trois desdits substituants peut être sélectionné parmi un groupement (alkylène en C₁-C₄)-O-(alkyle en C₁-C₄), CF₃, OCF₃, CHO, (alkylène en C₁-C₄)-OH, cyano, bromo et iodo, chacun des groupements alkyles en C₁-C₄ qui précèdent contenant en option une liaison double ou triple.

7. Composé selon la revendication 1, où A représente N, CH ou CCH₃.

8. Composé selon la revendication 1 où, dans les composés répondant aux formules I-E, (R)ₙ représente un atome d'hydrogène, CH₃ ou C₂H₅.

9. Composé selon la revendication 1, qui répond à une formule sélectionnée dans le groupe consistant en :
I-G, où (R)₁ représente CH₃ ou C₂H₅ ; I-H, où (R)₁ représente H ou CH₃ ; I-L, où (R)₂ représente (R^{a}R^{b}) et où R^{a} et R^{b} représentent tous deux H ; I-M, où (R)₂ représente (R^{a}R^{b}) et où R^{a} et R^{b} représentent tous deux H ; I-V, où (R)₁ représente CH₃ ; I-W ; et I-X.

10. Composé selon la revendication 1, où B représente -CHR¹R², NHCHR¹R² ou -OCHR¹R².

11. Composition pharmaceutique indiquée dans le traitement, la prévention ou l'inhibition, chez un mammifère, (a) d'une maladie pour laquelle le traitement peut être produit ou facilité par un antagonisme du facteur de libération de la corticotrophine (CRF), c'est-à-dire qui est induite ou favorisée par le CRF, ou (b) d'une maladie sélectionnée parmi les suivantes : pathologies inflammatoires comme la polyarthrite rhumatoïde et l'ostéoarthrose, une douleur, l'asthme, le psoriasis et des allergies ; trouble anxieux généralisé ; panique ; phobies ; trouble obsessionnel-compulsif ; état de stress post-traumatique ; troubles du sommeil induits par le stress ; perception d'une douleur, comme dans la fibromyalgie ; dérèglements de l'humeur comme une dépression, y compris une dépression majeure, un épisode dépressif unique, une dépression récurrente, une dépression induite par des sévices durant l'enfance, les troubles de l'humeur associés au syndrome de tension prémenstruelle et une dépression du postpartum ; mélancolie; maladies à forme bipolaire ; cyclothymie ; syndrome de fatigue chronique ; céphalées par tension nerveuse ; cancer ; syndrome du côlon irritable, maladie de Crohn ; colopathie spasmodique ; iléus post-opératoire ; ulcère ; diarrhée ; fièvre provoquée par un stress ; infections au virus de l'immunodéficience humaine (VIH) ; maladies neurodégénératives comme la maladie d'Alzheimer, la maladie de Parkinson et la chorée de Huntington ; maladies gastro-intestinales ; troubles du comportement alimentaire comme l'anorexie et la boulimie nerveuse ; stress hémorragique ; dépendances à des produits chimiques et toxicomanies (par exemple une dépendance à l'alcool, à la cocaïne, à l'héroïne, aux benzodiazépines ou à d'autres drogues) ; syndrome de sevrage de drogues qui provoquent une accoutumance ou de l'alcool ; épisodes psychotiques en réaction à un stress ; syndrome euthyroïdien ; syndrome de sécrétion inappropriée d'ADH (hormone antidiurétique) ; obésité ; stérilité ; traumatismes crâniens ; traumatismes de la moelle épinière ; lésions neuronales ischémiques (par ex. ischémie cérébrale comme une ischémie cérébrale avec atteinte hippocampique) ; lésions neuronales excitotoxiques ; épilepsie ; accident vasculaire cérébral ; dysfonctionnements immunitaires, y compris les dysfonctionnements immunitaires résultant d'un stress (par ex. la maladie exsudative du porc, la fièvre des transports des bovins ou la fibrillation paroxystique du cheval, ainsi que les dysfonctionnements induits par la claustration chez les volailles, par le stress associé à la tonte chez les ovins ou le stress lié aux interactions avec les humains chez le chien) ; spasmes musculaires ; incontinence urinaire ; démence sénile du type Alzheimer ; démence artériopathique ; sclérose latérale amyotrophique ; hypertension ; tachycardie ; insuffisance cardiaque congestive ; ostéoporose ; naissance avant-terme ; et hypoglycémie, ladite composition contenant une quantité d'un composé selon la revendication 1 qui est efficace dans le traitement d'une maladie de ce type et un véhicule pharmaceutiquement acceptable.

12. Utilisation d'un composé selon la revendication 1 dans la fabrication d'un médicament indiqué dans le traitement, la prévention ou l'inhibition, chez un mammifère, d'une maladie sélectionnée parmi les suivantes : pathologies inflammatoires comme la polyarthrite rhumatoïde et l'ostéoarthrose, une douleur, l'asthme, le psoriasis et des allergies ; trouble anxieux généralisé ; panique ; phobies ; trouble obsessionnel-compulsif ; état de stress post-traumatique ; troubles du sommeil induits par le stress ; perception d'une douleur, comme dans la fibromyalgie ; dérèglements de l'humeur comme une dépression, y compris une dépression majeure, un épisode dépressif unique, une dépression récurrente, une dépression induite par des sévices durant l'enfance, les troubles de l'humeur associés au syndrome de tension prémenstruelle et une dépression du postpartum ; mélancolie; maladies à forme bipolaire ; cyclothymie ; syndrome de fatigue chronique ; céphalées par tension nerveuse ; cancer ; syndrome du côlon irritable, maladie de Crohn ; colopathie spasmodique ; iléus post-opératoire ; ulcère ; diarrhée ; fièvre provoquée par un stress ; infections au virus de l'immunodéficience humaine (VIH) ; maladies neurodégénératives comme la maladie d'Alzheimer, la maladie de Parkinson et la chorée de Huntington ; maladies gastro-intestinales ; troubles du comportement alimentaire comme l'anorexie et la boulimie nerveuse ; stress hémorragique ; dépendances à des produits chimiques et toxicomanies (par exemple une dépendance à l'alcool, à la cocaïne, à l'héroïne, aux benzodiazépines ou à d'autres drogues) ; syndrome de sevrage de drogues qui provoquent une accoutumance ou de l'alcool ; épisodes psychotiques en réaction à un stress ; syndrome euthyroïdien ; syndrome de sécrétion inappropriée d'ADH (hormone antidiurétique) ; obésité ; stérilité ; traumatismes crâniens ; traumatismes de la moelle épinière ; lésions neuronales ischémiques (par ex. ischémie cérébrale comme une ischémie cérébrale avec atteinte hippocampique) ; lésions neuronales excitotoxiques ; épilepsie ; accident vasculaire cérébral ; dysfonctionnements immunitaires, y compris les dysfonctionnements immunitaires résultant d'un stress (par ex. la maladie exsudative du porc, la fièvre des transports des bovins ou la fibrillation paroxystique du cheval, ainsi que les dysfonctionnements induits par la claustration chez les volailles, par le stress associé à la tonte chez les ovins ou le stress lié aux interactions avec les humains chez le chien) ; spasmes musculaires ; incontinence urinaire ; démence sénile du type Alzheimer ; démence artériopathique ; sclérose latérale amyotrophique ; hypertension ; tachycardie ; insuffisance cardiaque congestive ; ostéoporose ; naissance avant-terme ; et hypoglycémie.

13. Composition pharmaceutique indiquée dans le traitement ou la prévention d'un trouble ou d'un état pathologique pour lequel le traitement ou la prévention peut être produit ou facilité par une inhibition de la protéine qui se lie à l'hormone libérant la corticotrophine (CRH) chez un mammifère, contenant une quantité d'un composé selon la revendication 1 capable d'inhiber la protéine qui se lie à la CRH et un véhicule pharmaceutiquement acceptable.

14. Composé selon la revendication 8 ou la revendication 9, où A représente N ou CH, R³ représente un groupement méthyle et (R)ₙ représente indépendamment un atome d'hydrogène ou un groupement méthyle.

15. Composé selon la revendication 14, où R⁵ représente un groupement phényle di- ou trisubstitué, dans lequel les deux ou les trois substituants sont indépendamment sélectionnés parmi un groupement fluoro, chloro, alkyle en C₁-C₄ et O-(alkyle en C₁-C₄) et un des deux ou trois desdits substituants peut être sélectionné parmi un groupement (alkylène en C₁-C₄)-O-(alkyle en C₁-C₄), CF₃, OCF₃, CHO, (alkylène en C₁-C₄) -OH, cyano, bromo et iodo, chacun des groupements alkyles en C₁-C₄ qui précèdent contenant en option une liaison double ou triple.

16. Composé selon la revendication 1, où ledit composé est l'un des suivants :
7-(1-Éthyl-propoxy)-5-méthyl-3-(2,4,6-triméthyl-phényl)-pyrazolo[1,5-a]pyrimidine ;
[2,5-Diméthyl-3-(2,4,6-triméthyl-phényl)-pyrazolo[1,5-a]pyrimidin-7-yl]-(1-éthylpropyl)-amine ;
(1-Éthyl-propyl)-[5-méthyl-3-(2,4,6-triméthyl-phényl)-pyrazolo[1,5-a]pyrimidin-7-yl]-amine ;
7-(1-Éthyl-propoxy)-2,5-diméthyl-3-(2,4,6-triméthyl-phényl)-pyrazolo[1,5-a]pyrimidine ;
[2,5-Diméthyl-3-(2,4,6-triméthyl-phényl)-pyrazolo[1,5-a]pyrimidin-7-yl]-éthyl-propylamine ;
ou un sel pharmaceutiquement acceptable desdits composés.
